# EUROPÄISCHE PATENTSCHRIFT

(11) **EP 3 895 642 B1**
(45) Veröffentlichungstag und Bekanntmachung des Hinweises auf die Patenterteilung: **27.09.2023**
(21) Anmeldenummer: 20169436.1
(22) Anmeldetag: 14.04.2020
(51) Int. Cl.: A61B 18/12, A61B 18/14, A61B 18/00

(54) **VORRICHTUNG ZUR KOAGULATION UND/ODER DISSEKTION VON BIOLOGISCHEM GEWEBE UND EIN VERFAHREN ZUM BETREIBEN EINER VORRICHTUNG**
DEVICE FOR COAGULATION AND/OR DISSECTION OF BIOLOGICAL TISSUE AND A METHOD OF OPERATING A DEVICE.
DISPOSITIF DE COAGULATION ET/OU DE DISSECTION DES TISSUS BIOLOGIQUES ET UNE MÉTHODE DE FONCTIONNEMENT D'UN DISPOSITIF.

(43) Veröffentlichungstag der Anmeldung: 20.10.2021
(73) Patentinhaber: Erbe Elektromedizin GmbH, 72072 Tübingen (DE)
(72) Erfinder: RIPPLINGER, Thomas, 72072 Tuebingen (DE)
(74) Vertreter: Rüger Abel Patentanwälte PartGmbB

(56) Entgegenhaltungen:
- EP-A1- 2 992 849
- EP-A1- 3 132 765
- WO-A1-02/100283

## Beschreibung

Die Erfindung betrifft eine Vorrichtung zur Koagulation und/oder Dissektion von biologischen Gewebe und ein Verfahren zum Betreiben einer Vorrichtung. Die Vorrichtung weist ein Instrument und ein das Instrument mit elektrischer Energie versorgendes Gerät auf.

Eine solche Vorrichtung ist beispielsweise aus EP 3 132 765 A1 bekannt. Das Instrument hat ein Werkzeug mit einer Schneidelektrode und mehreren Koagulationselektroden. Mittels einer Betriebsschaltung können die Elektroden mit elektrischer Energie versorgt werden. Hierzu weist die Betriebsschaltung einen Transformator auf, der sekundärseitig über einen Leistungsschalter mit der Schneidelektrode verbunden ist. Das Instrument hat einen Schneidaktivierungsschalter und einen Koagulationsaktivierungsschalter. Der Koagulationsaktivierungsschalter ist mechanisch mit dem Leistungsschalter gekoppelt. Beim Betätigen des Koagulationsaktivierungsschalters wird die elektrische Verbindung zwischen der Schneidelektrode und dem Transformator getrennt oder umgeschaltet, so dass keine Schneidspannung an der Schneidelektrode anliegt.

WO 2019/126370 A1 beschreibt eine Vorrichtung und ein Verfahren zur Koagulation und Dissektion von biologischem Gewebe. Die Vorrichtung hat ein Gerät mit einer Versiegelungsstufe und einer Dissektionsstufe zur elektrischen Versorgung eines angeschlossenen Instruments mit eine Koagulationsspannung zur Versiegelung oder einer Schneidspannung zur Dissektion. Beide Stufen weisen jeweils einen Wandler zur Spannungsregelung auf und können unabhängig voneinander betrieben werden. Zur Dissektion wird zunächst in einem ersten Zeitintervall die Versiegelungsspannung und anschließend in einem zweiten Zeitintervall die Schneidspannung bereitgestellt.

WO 02/100283 A1 beschreibt eine elektrochirurgische Vorrichtung mit einem Generator und einem daran angeschlossenen Instrument. Das Instrument hat mehrere Schalter. Durch das Betätigen eines ersten Schalters wird das Instrument in einem Koagulationsmodus und durch das Betätigen eines zweiten Schalters wird das Instrument in einem Schneidmodus betrieben. Den Schaltern sind Signalkodiermittel zugeordnet, um zu erkennen, welcher Schalter betätigt wurde und ein entsprechendes Steuersignal an den Generator zu übermitteln.

Aus EP 2 992 849 A1 ist eine Vorrichtung mit einem Generator und einem daran angeschlossenen Instrument bekannt, wobei das Instrument gleichzeitig zum Koagulieren und Schneiden von Gewebe eingerichtet ist. Über den Generator werden gleichzeitig Versiegelungselektroden und eine Schneidelektrode des Instruments mit einer Spannung beaufschlagt, so dass durch das Instrument gehaltene Gewebe getrennt und benachbart zu der Trennstelle versiegelt wird.

Die zur Koagulation und Dissektion verwendeten Instrumente können Einweginstrumente oder Mehrweginstrumente sein. Insbesondere bei Einweginstrumenten spielen die Herstellungskosten eine große Rolle. Eine sichere Bedienung muss gewährleistet sein.

Es kann daher als Aufgabe der vorliegenden Erfindung angesehen werden, ein Instrument zu schaffen, das einfach und sicher zu bedienen ist und sich kostengünstig herstellen lässt.

Diese Aufgabe wird durch eine Vorrichtung mit den Merkmalen des Patentanspruches 1 und ein Verfahren mit den Merkmalen des Patentanspruches 16 gelöst.

Die erfindungsgemäße Vorrichtung weist ein Gerät und ein elektrisch mit dem Gerät verbundenes Instrument auf. Mittels des Instruments kann biologisches Gewebe versiegelt (Koagulation) und/oder durchtrennt (Dissektion) werden. Dazu weist das Instrument entsprechende Elektroden auf.

Das Gerät ist dazu eingerichtet, an einem Geräteausgang eine Versorgungsspannung oder einen Versorgungsstrom für das Instrument bereitzustellen. Außerdem hat das Gerät eine Auswerteschaltung, die dazu eingerichtet ist, an einem zweiten Geräteausgang ein Auswertesignal für das Instrument bereitzustellen.

Das Instrument hat eine Betriebsschaltung mit einem Versorgungsanschluss zur Verbindung mit dem ersten Geräteausgang und mit einem Signalanschluss zur Verbindung mit dem zweiten Geräteausgang. Eine Steuerschaltung ist elektrisch mit dem Signalanschluss verbunden und dazu eingerichtet, ein Ansteuersignal zu erzeugen, das von einer Polarität des vom Gerät bereitgestellten Auswertesignals abhängt. Die Steuerschaltung hat außerdem einen ersten Schalter, der manuell bedienbar ist. Der erste Schalter dient beispielsgemäß zur Aktivierung des Dissektionsmodus der Vorrichtung.

Die Betriebsschaltung des Instruments weist außerdem eine mittels des Ansteuersignals ansteuerbare Umschalteinheit auf. Die Umschalteinheit kann einen ersten Schaltzustand oder einen zweiten Schaltzustand einnehmen. Die Umschalteinheit ist mittelbar oder unmittelbar mit dem Versorgungsanschluss und mittelbar oder unmittelbar mit dem Schneidausgang verbunden. Abhängig von der Polarität des Auswertesignals wird ein Ansteuersignal generiert, das die Umschalteinheit entweder dazu veranlasst, den ersten oder den zweiten Schaltzustand einzunehmen.

Bei einem bevorzugten Ausführungsbeispiel kann die Umschalteinheit in Reihe zwischen dem Versorgungsanschluss und dem Schneidausgang angeordnet sein. Bei dieser Ausgestaltung kann die Umschalteinheit beispielsweise zwischen einem leitenden ersten Schaltzustand und einem sperrenden zweiten Schaltzustand umgeschaltet werden. Alternativ kann die Umschalteinheit auch einen leitenden ersten und einen leitenden zweiten Schaltzustand aufweisen, wobei am Schneidausgang abhängig vom Schaltzustand entweder eine Koagulationsspannung oder eine Schneidspannung anliegt, je nachdem ob das Instrument bzw. die Vorrichtung im Koagulationsmodus oder Schneidmodus betrieben werden soll.

Im ersten Schaltzustand der Umschalteinheit liegt am Schneidausgang eine elektrische Schneidspannung an oder es wird ein elektrischer Schneidstrom bereitgestellt die bzw. der zur Dissektion von biologischem Gewebe geeignet ist. Demgegenüber ist am Schneidausgang im zweiten Schaltzustand keine elektrische Schneidspannung oder kein elektrischer Schneidstrom verfügbar, die bzw. der für eine Dissektion von biologischem Gewebe ausreichend ist. Im zweiten Schaltzustand kann am Schneidausgang entweder eine Spannung oder ein Strom etwa gleich Null anliegen oder alternativ eine Spannung oder ein Strom bereitgestellt werden, der für die Dissektion nicht ausreichend ist, aber zum Beispiel für die Koagulation geeignet ist.

Die Steuerschaltung ist dazu eingerichtet, eine Eigenschaft des Auswertesignals abhängig vom Betätigungszustand des ersten Schalters einzustellen bzw. zu modifizieren. Beispielsweise kann es sich bei der Eigenschaft des Auswertesignals um einen Betrag, eine Amplitude, einen Mittelwert oder dergleichen handeln. Die Eigenschaft ist vorzugsweise unabhängig von der Polarität des Auswertesignals. Bei einem bevorzugten Ausführungsbeispiel kann beispielsweise der Betrag eines durch die Steuerschaltung fließenden Auswertestromes als Eigenschaft verwendet werden, die charakterisiert, ob der erste Schalter betätigt oder unbetätigt ist.

Das Gerät der Vorrichtung ist wiederum dazu eingerichtet, die von der Steuerschaltung des Instruments beeinflusste bzw. eingestellte Eigenschaft des Auswertesignals, beispielsweise den Betrag des Auswertesignalstromes, zu erfassen. Das Gerät kann dadurch erkennen, ob der erste Schalter zur Aktivierung des Schneidmodus betätigt ist oder unbetätigt ist. Abhängig von der erfassten Eigenschaft des Auswertesignals wird wiederum die Polarität des Auswertesignals durch die Auswerteschaltung eingestellt.

Durch die Polarität des Auswertesignals wird wiederum der Zustand des Ansteuersignals vorgegeben und gegebenenfalls verändert. Somit kann durch die Polarität des Auswertesignals das zugehörige Ansteuersignal erzeugt und dadurch die Umschalteinheit veranlasst werden, den ersten oder den zweiten Schaltzustand einzunehmen, so dass die Vorrichtung im gewünschten Koagulationsmodus oder Dissektionsmodus betrieben wird.

Die Signalverarbeitung mittels der Steuerschaltung und der Auswerteschaltung ist sehr einfach und lässt sich kostengünstig realisieren. Aufwendige mechanische Einrichtungen zur Kopplung zwischen der Umschalteinrichtung und der Steuerschaltung können entfallen. Die Ansteuerung erfolgt über das Ansteuersignal elektrisch und/oder optisch. Die Vorrichtung kann auf einfache Weise mit Standardbauelementen aufgebaut werden.

Bei einem Ausführungsbeispiel kann die Umschalteinheit einen oder mehrere Halbleiterschalter aufweisen oder dadurch gebildet sein. Zumindest können mechanische Schalter in der Umschalteinheit entfallen. Dies wiederum vermeidet Funken- oder Lichtbogenbildung beim Ändern des Schaltzustandes.

Das Instrument der Vorrichtung kann ein Einweginstrument oder ein Mehrweginstrument sein. In beiden Fällen kann das Instrument einfach aufgebaut und dadurch kostengünstig hergestellt werden.

Wenn die von der Steuerschaltung eingestellte Eigenschaft des Auswertesignals angibt, dass der erste Schalter betätigt wurde, kann das Auswertesignal während verschiedener Phasen unterschiedliche Polaritätszustände aufweisen. Vorzugsweise weist das Auswertesignal im Dissektionsmodus bei betätigtem erstem Schalter während einer ersten Phase ab dem Betätigen des ersten Schalters ausschließlich eine erste Polarität auf. Insbesondere kann der Betrag des Auswertesignals während der ersten Phase konstant sein.

Unmittelbar auf die erste Phase kann eine zweite Phase folgen, in der das Auswertesignal abwechselnd bzw. alternierend die erste Polarität und eine der ersten Polarität entgegengesetzte zweite Polarität aufweist. Die erste Polarität kann beispielsweise negativ und die zweite Polarität kann beispielsweise positiv sein oder umgekehrt.

Das Auswertesignal kann beispielsweise ein Strom oder eine Spannung sein. Während der ersten Phase kann beispielsweise ein Gleichstrom oder eine Gleichspannung als Auswertesignal vorliegen und während der zweiten Phase kann beispielsweise eine Wechselspannung oder ein Wechselstrom als Auswertesignal vorliegen. Die Signalform während der zweiten Phase kann variieren und es kann sich beispielsweise um ein Rechtecksignal, ein Dreiecksignal oder ein sinusförmiges Signal handeln. Während der zweiten Phase kann das Auswertesignal positive und negative Halbwellen aufweisen, die die gleiche Dauer oder unterschiedliche Dauern und/oder die gleiche Amplitude oder unterschiedliche Amplituden aufweisen können.

Während der zweiten Phase können die Signalanteile des Auswertesignals mit erster Polarität zur Auswertung des Betätigungszustands des ersten Schalters verwendet werden und die Signalanteile mit zweiter Polarität können dazu verwendet werden, mittels des Ansteuersignals die Umschalteinheit im gewünschten Schaltzustand zu halten.

Insbesondere ist die Steuerschaltung dazu eingerichtet, die Umschalteinheit mittels des Ansteuersignals in den ersten Schaltzustand umzuschalten, wenn das Auswertesignal während der zweiten Phase zumindest zeitweise die zweite Polarität aufweist. In diesem ersten Schaltzustand liegt am Schneidausgang eine elektrische Schneidspannung an bzw. es wird ein elektrischer Schneidstrom bereitgestellt.

Wenn das Auswertesignal in der ersten Phase lediglich die erste Polarität aufweist, wird die Umschalteinheit mittels des Ansteuersignals dazu veranlasst, den zweiten Schaltzustand einzunehmen. Das Ansteuersignal kann im Wesentlichen Null sein, wenn das Auswertesignal die erste Polarität aufweist. In diesem zweiten Schaltzustand ist am Schneidausgang keine Spannung und kein Strom verfügbar, der eine Dissektion von biologischem Gewebe ausführen kann.

Bei einer bevorzugten Ausführungsform kann das Gerät eine ansteuerbare Energiequelle (Stromquelle oder Spannungsquelle) aufweisen, um die Versorgungsspannung bzw. den Versorgungsstrom am ersten Geräteausgang bereitzustellen. Die Auswerteschaltung des Geräts kann dazu eingerichtet sein, die Energiequelle mittels eines Aktivierungssignals anzusteuern. Mittels des Aktivierungssignals kann die Versorgungsspannung bzw. den Versorgungsstrom beispielsweise eingeschaltet und ausgeschaltet werden.

Es ist vorteilhaft, wenn die Steuerschaltung zusätzlich zu dem ersten Schalter einen manuell betätigbaren zweiten Schalter aufweist. Der zweite Schalter kann zur Aktivierung eines Koagulationsmodus dienen. Bei einer Ausführungsform der Erfindung können die beiden Schalter so ausgeführt sein, dass sie unabhängig voneinander - also jeweils einzeln oder auch beide gleichzeitig - betätigbar sind. Alternativ dazu können die beiden Schalter auch mechanisch gekoppelt sein, so dass immer nur einer der beiden Schalter betätigt werden kann, wie es etwa bei einer Schaltwippe der Fall ist.

Bei einem bevorzugten Ausführungsbeispiel sind der erste Schalter und der zweite Schalter jeweils als Taster ausgebildet. Die Schalter können beispielsweise in ihrem Ruhezustand in einem sperrenden Zustand sein und manuell in einen leitenden Zustand umgeschaltet werden.

Es ist vorteilhaft, wenn die Steuerschaltung dazu eingerichtet ist, die Eigenschaft des Auswertesignals abhängig vom Betätigungszustand des zweiten Schalters einzustellen. Beispielsweise kann der Betrag des Auswertesignals abhängig davon variieren, ob keiner der Schalter, nur der erste Schalter, nur der zweite Schalter oder beide Schalter betätigt sind. Je nachdem welche Schalterbetätigung über die Eigenschaft des Auswertesignals in der Auswerteschaltung des Geräts erkannt wurde, wird dessen Polarität eingestellt. Bei einem Ausführungsbeispiel wird der Dissektionsmodus mit einer Phase wechselnder Polarität des Auswertesignals nur dann aktiviert, wenn ausschließlich der erste Schalter betätigt wurde. In allen anderen Fällen kann das Auswertesignal ausschließlich die erste Polarität aufweisen.

Es ist außerdem vorteilhaft, wenn die Auswerteschaltung dazu eingerichtet ist, das Aktivierungssignal zum Einschalten bzw. Bereitstellen der Versorgungsspannung oder des Versorgungsstromes am ersten Geräteausgang zu erzeugen, wenn mittels der Eigenschaft des Auswertesignals erkannt wurde, dass einer der beiden Schalter betätigt wurde .

Das Instrument kann bei einem Ausführungsbeispiel wenigstens eine Schneidelektrode, wenigstens eine erste Koagulationselektrode und wenigstens eine zweite Koagulationselektrode aufweisen. Beispielsweise kann der Schneidausgang mit der wenigstens einen Schneidelektrode verbunden sein. Ein erster Koagulationsausgang der Betriebsschaltung kann mit der wenigstens einen ersten Koagulationselektrode verbunden sein. Ein zweiter Koagulationsausgang der Betriebsschaltung kann mit der wenigstens einen zweiten Koagulationselektrode verbunden sein.

Bei einer bevorzugten Ausführungsform weist die Steuerschaltung ein Ansteuerelement auf, das zur Erzeugung des Ansteuersignals eingerichtet ist.

Es ist vorteilhaft, wenn die Betriebsschaltung eine Kopplungseinrichtung aufweist, die ein Senderbauteil und ein galvanisch vom Senderbauteil getrenntes Empfängerbauteil aufweist. Über das Senderbauteil kann das Ansteuersignal von der Steuerschaltung zum Empfängerbauteil übertragen werden, das mit der Umschalteinheit verbunden ist oder zur Umschalteinheit gehört.

Bei einem Ausführungsbeispiel ist das Senderbauteil durch das Ansteuerelement gebildet. Dabei kann das Empfängerbauteil mit einem Steuereingang eines ansteuerbaren Halbleiterschalters der Umschalteinrichtung verbunden sein.

Als Senderbauteil kann beispielsweise wenigstens eine Leuchtdiode und als Empfängerbauteil wenigstens eine Fotodiode oder wenigstens eine Fototransistor verwendet werden. Beispielsweise kann das Senderbauteil und das Empfängerbauteil als gemeinsame Baugruppe in einem gemeinsamen Gehäuse angeordnet werden. Diese Baugruppe kann beispielsweise ein Optokoppler sein.

Es ist vorteilhaft, wenn das Empfängerbauteil über eine Lade- und Entladeschaltung mit dem wenigstens einen Steuereingang der Umschalteinheit verbunden ist. Mittels der Lade- und Entladeschaltung ist es möglich den ersten Schaltzustand der Umschalteinheit bei einer Dissektionsanforderung (z.B. Betätigen des ersten Schalters) auch während der Zeitdauer aufrechtzuerhalten, während der eine erste Halbwelle anliegt. Vorzugsweise ist die Lade- und Entladeschaltung auch dazu eingerichtet, die elektrischen Ladungen im wenigstens einen Steuereingang der Umschalteinheit abzubauen, wenn keine zur Dissektion geeignete Spannung an die Schneidelektrode angelegt werden soll, um ein Umschalten der Umschalteinheit in den zweiten Schaltzustand zu ermöglichen.

Die Umschalteinheit kann bei einem Ausführungsbeispiel ohne mechanische Schalter ausgebildet sein und ausschließlich Halbleiterschalter aufweisen, wie etwa Bipolartransistoren und/oder Feldeffekttransistoren und/oder IGBTs.

Es ist vorteilhaft, wenn der erste Schalter und das Ansteuerelement in einem ersten Schaltungszweig in Reihe geschaltet sind. Der erste Schaltungszweig kann mit dem Signalanschluss verbunden sein. Dadurch kann ein Stromfluss durch das Ansteuerelement bei geöffnetem ersten Schalter unterbunden werden.

Beispielsweise kann im ersten Schaltungszweig ein Einbahnstrompfad vorhanden sein, in dem das Ansteuerelement angeordnet ist. In dem Einbahnstrompfad wird der Stromfluss nur in eine Richtung zugelassen, insbesondere in die Stromflussrichtung bei anliegender zweiter Halbwelle des Auswertesignals. In dem Einbahnstrompfad ist dazu wenigstens ein Bauteil mit Diodenfunktion vorhanden. Das Bauteil mit Diodenfunktion kann beispielsweise das Ansteuerelement selbst sein. Alternativ oder zusätzlich kann ein weiteres Bauteil mit Diodenfunktion, im einfachsten Fall eine Diode, im Einbahnstrompfad vorhanden sein. Alternativ zur Diode können auch gesteuerte Halbleitschalter verwendet werden, die nur bei einer zweiten Halbwelle des Auswertesignals ihren leitenden Zustand einnehmen.

Bei einem Ausführungsbeispiel ist das Ansteuerelement als Leuchtdiode ausgeführt. Zusätzlich zu der Leuchtdiode kann im Einbahnstrompfad eine separate Diode in Reihe zum Ansteuerelement geschaltet sein, vorzugsweise in Stromflussrichtung stromaufwärts.

Es ist vorteilhaft, wenn im ersten Schaltungszweig parallel zum Einbahnstrompfad ein weiterer Parallelstrompfad vorhanden ist, indem beispielsweise ein Widerstand angeordnet sein kann. Wenn in der vorliegenden Anmeldung von einem Widerstand die Rede ist, ist ein Ohmscher Widerstand gemeint, solange nichts anderes angegeben ist. Der erste Schalter ist vorzugsweise in Reihe zum Einbahnstrompfad und zum Parallelstrompfad geschaltet.

Es ist außerdem vorteilhaft, wenn der zweite Schalter in einem zweiten Schaltungszweig der Steuerschaltung angeordnet ist. Der zweite Schaltungszweig ist mit dem Signalanschluss verbunden. Vorzugsweise ist in Reihe zum zweiten Schalter ein Widerstand geschaltet.

Bei einem Ausführungsbeispiel sind der zweite Schalter und das Ansteuerelement über einen Verbindungsstrompfad gekoppelt. Beispielsweise kann der Verbindungsstrompfad eine permanente, nicht sperrbare elektrische Verbindung zwischen dem zweiten Schalter und dem Ansteuerelement herstellen. Der Verbindungsstrompfad kann bauteilfrei ausgebildet sein. Insbesondere sind der zweite Schalter und der Verbindungsstrompfad parallel zum Ansteuerelement und dem ersten Schalter geschaltet. Bei geschlossenem bzw. leitendem zweiten Schalter wird eine niederohmige Überbrückung des Ansteuerelements bewirkt. Bei leitendem zweiten Schalter kann somit kein Stromfluss durch das Ansteuerelement erfolgen. Unter einer niederohmigen Überbrückung des Ansteuerelements ist eine Überbrückung zu verstehen, die einen derart geringen Widerstandswert hat, dass der Spannungsabfall an der Überbrückung im Wesentlichen gleich null ist und insbesondere kleiner ist als eine Aktivierungsspannung des Ansteuerelements, beispielsweise die Flussspannung einer Leuchtdiode.

Es ist außerdem vorteilhaft, wenn der erste Koagulationsausgang der Betriebsschaltung schalterlos mit dem Versorgungsanschluss verbunden ist. In dieser Verbindung kann beispielsweise ein Kondensator angeordnet sein.

Vorzugsweise hat die Betriebsschaltung eine Transformatorschaltung mit einem Transformator. Die Transformatorschaltung ist primärseitig mit dem Versorgungsanschluss und sekundärseitig mit dem Schneidausgang verbunden, wobei diese Verbindungen mittelbar oder unmittelbar sein können. Der Transformator der Transformatorschaltung kann ohne galvanische Trennung als Spartransformator ausgeführt sein. Der Transformator kann alternativ auch eine von der Sekundärseite galvanisch getrennte Primärseite aufweisen. Die Transformatorschaltung ist insbesondere dazu eingerichtet, die am Versorgungsanschluss anliegende Versorgungsspannung zu erhöhen, beispielsweise von etwa 100 Volt auf etwa 450 Volt Wechselspannung.

Bei den vorstehend erläuterten Ausführungsbeispielen ist die Betriebsschaltung Bestandteil des Instruments und kann in einem Gehäuse des Instruments angeordnet sein. Die Betriebsschaltung oder Teile davon können zusätzlich oder alternativ zu der Unterbringung im Gehäuse des Instruments auch in einem separaten Modul angeordnet sein, das mit dem Instrument elektrisch verbindbar ist und beispielsweise elektrisch zwischen das Gerät und das Instrument geschaltet werden kann. Das Modul kann beispielsweise ein Buchsenmodul sein, das elektrisch und/oder mechanisch lösbar mit dem Gerät und dem Instrument verbindbar ist. Es kann auch in der Anschlussleitung bzw. dem Kabel des Instruments angeordnet sein.

Ein weiterer unabhängiger Aspekt der Erfindung ist gekennzeichnet durch ein elektrochirurgisches Instrument, das dazu eingerichtet ist, biologisches Gewebe in einem Koagulationsmodus zu versiegeln und in einem Dissektionsmodus zu durchtrennen. Das Instrument hat eine elektronisch und/oder optisch ansteuerbare Umschalteinheit, die wenigstens einen Halbleiterschalter aufweist. Die Umschalteinheit kann in einem ersten Schaltzustand an einer Schneidelektrode eine zur Dissektion von biologischem Gewebe geeignete elektrische Größe (Schneidspannung und/oder Schneidstrom) bereitstellen. Demgegenüber ist in einem zweiten Schaltzustand der Umschalteinheit keine elektrische Schneidspannung oder kein elektrischer Schneidstrom an der Schneidelektrode verfügbar, die bzw. der für eine Dissektion von biologischem Gewebe ausreichend ist. Im Dissektionsmodus erfolgt die Ansteuerung der Umschalteinheit derart, dass die Umschalteinheit zumindest während einer Phase und vorzugsweise vor Beginn der Dissektion, den zweiten Schaltzustand einnimmt. Im Koagulationsmodus bleibt die Umschalteinheit dauerhaft im zweiten Schaltzustand. Ein elektrisches und/oder optische Ansteuersignal zur Ansteuerung der Umschalteinheit kann durch das Instrument erzeugt werden, beispielsweise basierend auf dem vorstehend erläuterten Auswertesignal und/oder einem anderen Signal und/oder einem durch das Instrument erfassbaren Zustand eines Geräts zur elektrischen Versorgung des Instruments, an das das Instrument angeschlossen ist, oder alternativ dazu auch unabhängig vom Gerät.

Dieser weitere Aspekt kann mit einem oder mehreren Merkmalen der vorstehend erläuterten Vorrichtung und insbesondere der Ausgestaltung des vorstehend beschriebenen Instruments gemäß dem zuerst geschilderten Aspekt der Erfindung kombiniert werden.

Ein Verfahren zum Betreiben einer Vorrichtung, insbesondere unter Verwendung eines der vorstehend beschriebenen Ausführungsbeispiele, weist folgende Schritte auf:
Zunächst wird ein Auswertesignal erzeugt und am zweiten Geräteausgang für das Instrument und insbesondere die Steuerschaltung bereitgestellt. In einem Ausgangszustand weist das Auswertesignal bevorzugt lediglich die erste Polarität auf.

Das Auswertesignal wird am Signalanschluss empfangen. Abhängig von dem Betätigungszustand des ersten Schalters und des optional vorhandenen zweiten Schalters wird eine Eigenschaft des Auswertesignals verändert, die durch die Auswerteschaltung detektierbar ist. Die von der Auswerteschaltung erfasste Eigenschaft des Auswertesignals kann beispielsweise der Betrag eines aufgrund des Auswertesignals durch die Steuerschaltung fließenden Auswertestromes sein. Diese Eigenschaft gibt an, ob der erste Schalter bzw. der optional vorhandene zweite Schalter betätigt wurden und somit ob die Vorrichtung im Dissektionsmodus (z.B. erster Schalter betätigt) oder im Koagulationsmodus (z.B. zweiter Schalter betätigt) betrieben werden soll. Die Polarität des Auswertesignals wird abhängig von der erfassten Eigenschaft des Auswertesignals und mithin abhängig vom Betätigungszustand des wenigstens einen Schalters eingestellt.

Aufgrund der eingestellten Polarität des Auswertesignals wird wiederum das Ansteuersignal für die Umschalteinheit erzeugt, so dass diese entweder zur Einnahme des ersten Schaltzustandes oder zur Einnahme des zweiten Schaltzustandes veranlasst wird.

Vorteilhafte Ausgestaltungen der Erfindung ergeben sich aus den abhängigen Patentansprüchen, der Beschreibung und der Zeichnungen. Nachfolgend werden bevorzugte Ausführungsbeispiele der Erfindung anhand der beigefügten Zeichnungen im Einzelnen erläutert. Es zeigen:
Figur 1 eine schematische Darstellung einer Vorrichtung 20 zur Koagulation und Dissektion mit einem Gerät und einem elektrisch mit dem Gerät verbundenen Instrument,
Figur 2 eine perspektivische Teildarstellung eines Werkzeugs des Instruments aus Figur 1,
Figur 3 einen Schaltplan eines Ausführungsbeispiels einer Betriebsschaltung des Instruments,
Figur 4 eine schematische Darstellung eines zeitlichen Verlaufs eines beispielhaften Auswertesignals,
Figur 5 eine schematische Darstellung eines zeitlichen Verlaufs einer Koagulationsspannung in einem Dissektionsmodus der Vorrichtung,
Figur 6 eine schematische Darstellung eines zeitlichen Verlaufs einer Schneidspannung in einem Dissektionsmodus der Vorrichtung,
Figuren 7-12 einen Schaltplan eines Ausführungsbeispiels einer Steuerschaltung der Betriebsschaltung aus Figur 3 in unterschiedlichen Zuständen,
Figur 13 einen Schaltplan einer optionalen Spannungserhöhungsschaltung für die Betriebsschaltung aus Figur 3,
Figur 14 einen Schaltplan für ein abgewandeltes Ausführungsbeispiel für eine Umschalteinheit für die Betriebsschaltung aus Figur 3, und
Figur 15 ein abgewandeltes Ausführungsbeispiel eines Schaltungsteils der Steuerschaltung zur Realisierung einer Diodenfunktion mittels eines Transistors.

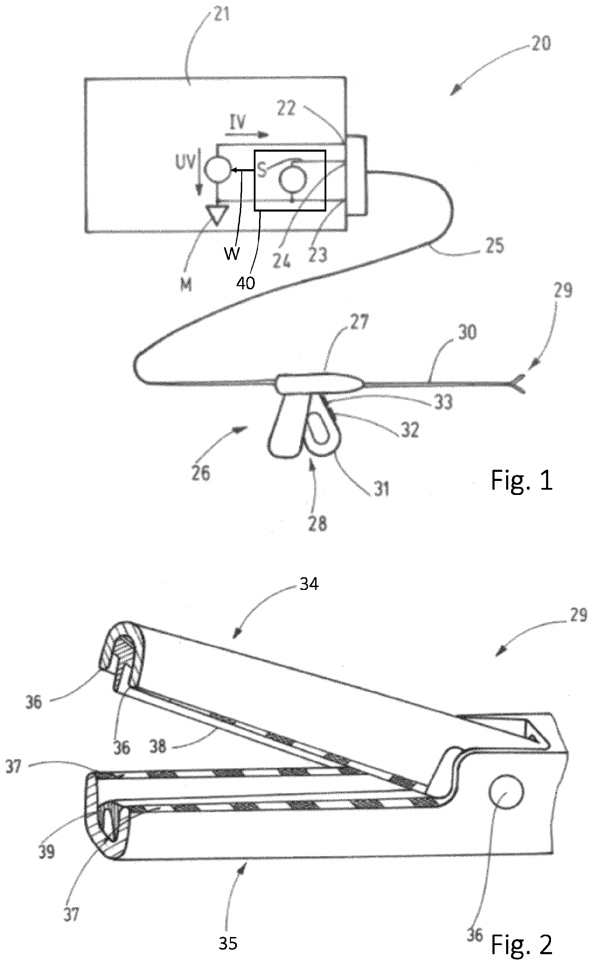

In Figur 1 ist eine Vorrichtung 20 zur Koagulation und Dissektion veranschaulicht. Die Vorrichtung 20 hat ein Gerät 21, das an einem ersten Geräteausgang 22 eine Versorgungsspannung UV oder einen Versorgungsstrom IV bereitstellt. Dazu weist das Gerät eine vorzugsweise mittels eines Aktivierungssignals W ansteuerbare oder zumindest ein- und ausschaltbare HF-Stromquelle oder HF-Spannungsquelle 42 auf. An einem zweiten Geräteausgang 24 wird ein Auswertesignal S bereitgestellt. Das Auswertesignal S wird von einer Auswerteschaltung 40 des Geräts 21 erzeugt. Ein dritter Geräteausgang 23 ist mit Masse M verbunden.

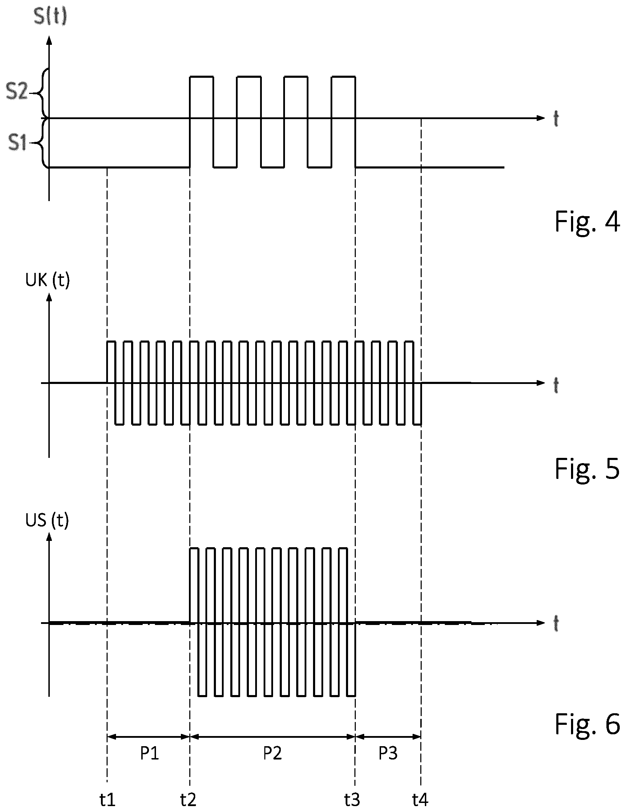

Das Auswertesignal S ist beispielsweise eine Spannung oder ein Strom. Es kann eine erste Polarität S1 oder eine der ersten Polarität S1 entgegengesetzte zweite Polarität annehmen. Die erste Polarität ist beispielsgemäß negativ und die zweite Polarität positiv. Phasenweise kann die Polarität S1, S2 des Auswertesignals S konstant sein und phasenweise alternierend (Figur 4). Das Auswertesignal S ist in Figur 4 als Rechtecksignal veranschaulicht. Alternativ dazu könnte das Auswertesignal S in einer Phase mit alternierender Polarität S1, S2 auch irgendwelche anderen Wellenformen aufweisen, beispielsweise eine sinusförmige oder eine dreieckförmige Wellenform mit positiven und negativen Halbwellen. Während einer Phase mit wechselnder Polarität S1, S2 kann das Auswertesignal S periodisch oder aperiodisch sein. Es ist außerdem möglich, dass eine Zeitdauer für eine Halbwelle mit erster Polarität und eine Zeitdauer für eine Halbwelle mit zweiter Polarität unterschiedlich lang sind.

Vorzugsweise hat das Auswertesignal S in einem Ausgangszustand ausschließlich eine Polarität, beispielsgemäß die erste Polarität S1. Dabei kann das Auswertesignal einen konstanten Betrag aufweisen.

An das Gerät 21 ist über ein mehradriges Kabel 25 ein Instrument 26 zur Koagulation und Dissektion angeschlossen. Das Instrument 26 hat ein Gehäuse 27 mit Handgriff 28 sowie ein Werkzeug 29. Das Werkzeug 29 ist beim Ausführungsbeispiel über ein Verbindungsteil 30 mit dem Gehäuse 27 verbunden. Das Verbindungsteil 30 kann stabförmig ausgeführt sein. An dem Handgriff 28 ist ein Bedienelement 31 für das Werkzeug 29 vorhanden. Das Bedienelement 31 dient zur mechanischen und elektrischen Betätigung des Werkzeugs 29. An dem Bedienteil 28 ist ein manuell betätigbarer erster Schalter 32 sowie ein manuell betätigbarer zweiter Schalter 33 vorhanden. Die beiden Schalter 32, 33 sind beim Ausführungsbeispiel als Taster ausgebildet und befinden sich in ihrem nicht betätigten Ruhezustand in einem elektrisch sperrenden Zustand.

In Figur 2 ist das Werkzeug 29 des Instruments 26 aus Figur 1 in einer perspektivischen Teildarstellung veranschaulicht. Das Werkzeug 29 hat zwei relativ zueinander bewegbare Branchen 34, 35, die mittels eines Gelenks 41 gelenkig miteinander verbunden sind. Über das Bedienelement 31 können die Branchen 34, 35 relativ zueinander geschwenkt werden. An der einen Branche 34 ist wenigstens eine erste Koagulationselektrode 36 angeordnet, während an der jeweils anderen Branche 35 wenigstens eine zweite Koagulationselektrode 37 vorhanden ist. Jeweils eine erste Koagulationselektrode 36 und eine zugeordnete zweite Koagulationselektrode 37 bilden ein Koagulationselektrodenpaar. An den Branchen 34, 35 können mehrere Koagulationselektrodenpaare vorhanden sein.

Außerdem verfügt eine der Branchen 34 zusätzlich über eine Schneidelektrode 38. Die Schneidelektrode 38 ist beim Ausführungsbeispiel als Einsatz in einer nutförmigen Vertiefung an der Branche 35 angeordnet und durch zwei erste Koagulationselektroden 36 flankiert. Die Schneidelektrode 38 ist von den ersten Koagulationselektroden 36 elektrisch isoliert am Werkzeug 39 angeordnet. Die mehreren ersten Koagulationselektroden 36 können elektrisch miteinander verbunden sein.

Der Branche 34 ist ein Widerlager 39 für die Schneidelektrode 38 angeordnet. Wird das Werkzeug 29 über das Bedienelement 31 geschlossen, liegen sich jeweils eine erste Koagulationselektrode 36 und eine zweite Koagulationselektrode 37 gegenüber. Die Schneidelektrode 38 liegt benachbart oder am Widerlager 39 an. Über die Schalter 32, 33 können die elektrischen Funktionen der Elektroden 36, 37, 38 bedient werden.

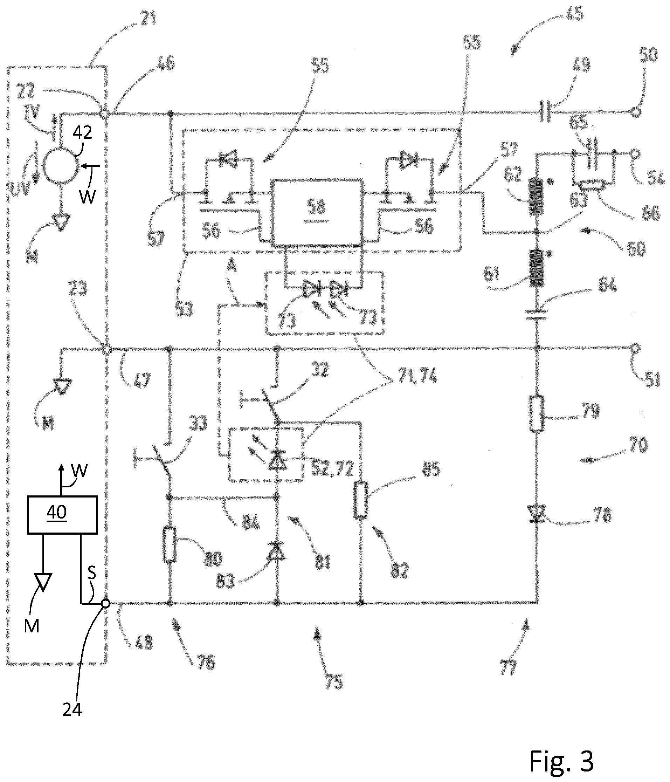

Ein Ausführungsbeispiel einer Betriebsschaltung 45 des Instruments 26 ist in Figur 3 veranschaulicht. Die Betriebsschaltung 45 hat einen Versorgungsanschluss 46, mittels dem die Betriebsschaltung 45 an den ersten Geräteausgang 22 anschließbar ist sowie einen Masseanschluss 47, mittels dem die Betriebsschaltung 45 mit dem dritten Geräteausgang 23 verbindbar ist. Ein Signalanschluss 48 der Betriebsschaltung 45 ist mit dem zweiten Geräteausgang 24 verbindbar. Am Signalanschluss 48 liegt daher das Auswertesignal S des Geräts 21 an. Über den Masseanschluss 47 wird die Betriebsschaltung 45 mit Masse M verbunden. Der Versorgungsanschluss 46 wird beispielsgemäß an die HF-Stromquelle oder HF-Spannungsquelle 42 des Geräts 21 angeschlossen, um einen Versorgungsstrom IV oder eine Versorgungsspannung UV für die Betriebsschaltung 45 bereitzustellen.

Der Versorgungsanschluss 46 ist über einen ersten Kondensator 49 mit einem Koagulationsausgang 50 verbunden. Der erste Koagulationsausgang 50 ist mit der wenigstens einen ersten Koagulationselektrode 36 verbunden. Ein zweiter Koagulationsausgang 51 ist mit der wenigstens einen zweiten Koagulationselektrode 37 verbunden. Der zweite Koagulationsausgang 51 ist beim Ausführungsbeispiel als Masseausgang ausgeführt. Der zweite Koagulationsausgang 51 ist dazu mit dem Masseanschluss 47 uns mithin mit der Masse M verbunden.

Die Betriebsschaltung 45 weist außerdem eine mittels eines Ansteuersignals A eines Ansteuerelements 52 umschaltbare Umschalteinheit 53 auf. Die Umschalteinheit 53 ist in die elektrische Verbindung zwischen dem Versorgungsanschluss 46 und einem Schneidausgang 54 geschaltet. Die Umschalteinheit 53 ist dazu eingerichtet, am Schneidausgang 54 in einem ersten Schaltzustand eine Schneidspannung US und/oder einen Schneidstrom bereitzustellen, der für eine Dissektion unter Verwendung der Schneidelektrode 38 eingerichtet ist und in einem zweiten Schaltzustand an dem Schneidausgang 54 im Wesentlichen keine elektrische Energie bereitzustellen. Im zweiten Schaltzustand wird beispielsgemäß die Verbindung zwischen dem Versorgungsanschluss 46 und dem Schneidausgang 54 unterbrochen.

Die Umschalteinheit 53 ist ohne mechanische Schalter ausgeführt und weist wenigstens einen Halbleitschalter 55 und beim Ausführungsbeispiel zwei in Reihe geschaltete Halbleiterschalter 55 auf. Beispielsgemäß ist jeder Halbleiterschalter 55 durch einen Feldeffekttransistor gebildet, der hier als normal sperrender n-Kanal MOSFET veranschaulicht ist. Jeder Halbleiterschalter 55 hat einen Steueranschluss 56, der beim Ausführungsbeispiel durch das Gate der MOSFETS gebildet ist.

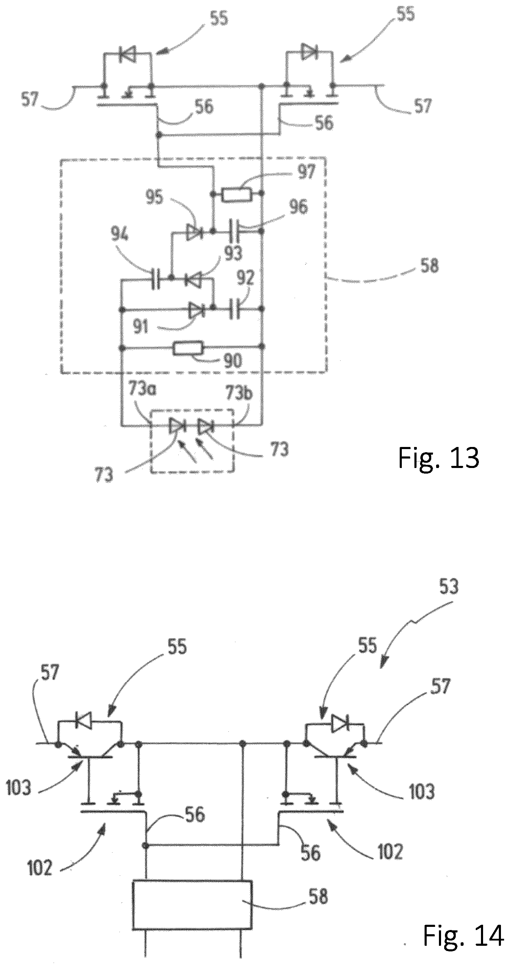

Die beiden Steueranschlüsse 56 sind vorzugsweise miteinander verbunden. Außerdem können die beiden Source-Anschlüsse der MOSFETS miteinander verbunden sein (Fig. 13) .

Die Drain-Anschlüsse der beiden MOSFETS bilden jeweils einen Schaltanschluss 57 der Umschalteinheit 53. Zwischen den beiden Schaltanschlüssen 57 ist die Schaltstrecke der Umschalteinheit 53 gebildet. Der eine Schaltanschluss 57 der Umschalteinheit 53 ist mit dem Versorgungsanschluss 46 verbunden. Der jeweils andere Schaltanschluss 57 ist mit dem Schaltausgang 54 verbunden.

Die Umschalteinheit 53 weist beispielsgemäß eine Lade- und Entladeschaltung 58 für den wenigstens einen Halbleitschalter 55 auf. Die Lade- und Entladeschaltung 58 ist dazu eingerichtet elektrische Ladungen an den Steueranschlüssen 56 ausreichend lange halten zu können, um den ersten Schaltzustand der Umschalteinheit 53 während einer Phase mit wechselnder Polarität S1, S2 des Auswertesignals S aufrechterhalten zu können, solange der erste Schalter 32 betätigt ist. Beispielsgemäß wird das Ansteuersignal A zum Aufrechterhalten des ersten Schaltzustandes nur dann erzeugt, wenn das Auswertesignal S eine der beiden Polaritäten S1 oder S2 und beispielsgemäß die zweite Polarität S2 aufweist. Dies führt dazu, dass in einer Phase mit wechselnder Polarität S1, S2 des Auswertesignals S auch das Ansteuersignal A seinen Zustand alternierend ändert. Während einer solchen Phase verhindert die Lade- und Entladeschaltung 58 das alternierende Umschalten der Umschalteinheit 53 zwischen dem ersten und zweiten Schaltzustand.

Zusätzlich ist die Lade- und Entladeschaltung 58 dazu eingerichtet, die an Steueranschlüssen 56 vorhandenen Ladungen abzuführen, wenn der erste Schalter 32 nicht mehr betätigt wird, so dass die Halbleiterschalter 55 auch wieder in ihren nicht leitenden Zustand übergehen können (entspricht beispielsgemäß dem zweiten Schaltzustand).

Die Betriebsschaltung 45 hat beim Ausführungsbeispiel eine Transformatorschaltung mit einem Transformator 60. Der Transformator 60 weist eine Primärwicklung 61 und eine Sekundärwicklung 62 auf. Beim Ausführungsbeispiel ist der Transformator 60 als Spartransformator ausgebildet. Die Primärwicklung 61 und die Sekundärwicklung 62 sind dabei in Reihe geschaltet und ein Mittelabgriff 63 ist mit einem zugeordneten Schaltanschluss 57 der Umschalteinrichtung 53 verbunden. Ausgehend vom Mittelabgriff 63 ist eine Reihenschaltung aus der Primärwicklung 61 und einem zweiten Kondensator 64 mit Masse M verbunden. Ausgehend vom Mittelabgriff 63 ist eine Reihenschaltung aus der Sekundärwicklung 62 und einem dritten Kondensator 65 mit dem Schneidausgang 54 verbunden. Parallel zum dritten Kondensator 65 ist ein erster Widerstand 66 geschaltet. Die Parallelschaltung aus dem dritten Kondensator 65 und dem ersten Widerstand 66 bildet eine Funkenerkennungsschaltung. Treten Funken auf, hat der Versorgungsstrom IV bzw. die Versorgungsspannung UV einen Gleichanteil, was in dem Gerät 21 optional ausgewertet und erkannt werden kann. Ist eine solche Funkenerkennung nicht erforderlich, kann die Funkenerkennungsschaltung auch entfallen.

Die Betriebsschaltung 45 verfügt außerdem über eine Steuerschaltung 70, zu der das Ansteuerelement 52 gehört. Die Steuerschaltung 70 ist über eine Kopplungseinrichtung 71 mit der Umschalteinheit 53 gekoppelt, um das Ansteuersignal A von der Steuerschaltung 70 zu der Umschalteinheit 53 zu übertragen. Die Kopplungseinrichtung 71 hat hierfür wenigstens ein Senderbauteil 72 in der Steuerschaltung 70 und wenigstens ein Empfängerbauteil 73, das mit dem wenigstens einen Steueranschluss 56 der Umschalteinheit 53 verbunden ist. Beim Ausführungsbeispiel ist das wenigstens eine Senderbauteil 72 eine Leuchtdiode und das wenigstens eine Empfängerbauteil 73 eine Fotodiode oder alternativ ein Fototransistor. Die Kopplungseinrichtung 71 kann durch einen Optokoppler 74 gebildet sein. Bei dem hier gezeigten Ausführungsbeispiel ist das Senderbauteil 72 durch das Ansteuerelement 52 gebildet.

Die Steuerschaltung 70 weist bei dem in Figur 3 gezeigten Ausführungsbeispiel einen ersten Schaltungszweig 75 mit dem ersten Schalter 32, einen zweiten Schaltungszweig 76 mit dem zweiten Schalter 33 sowie einen dritten Schaltungszweig 77 auf. Die drei Schaltungszweige 75, 76, 77 sind parallel zueinander zwischen den Masseanschluss 47 und den Signalanschluss 48 geschaltet.

Der dritte Schaltungszweig 77 ist beim Ausführungsbeispiel durch eine Reihenschaltung aus einer ersten Diode 78 und einem zweiten Widerstand 79 gebildet. Die Kathode der ersten Diode 78 ist mit dem Signalanschluss 58 und die Anode mit dem zweiten Widerstand 79 verbunden. Der zweite Widerstand 79 ist mit seinem anderen Anschluss mit Masse M verbunden.

Der zweite Schaltungszweig 76 weist zusätzlich zum zweiten Schalter 33 einen dritten Widerstand 80 auf, der in Reihe zum zweiten Schalter 33 geschaltet ist.

Der erste Auswertepfad 75 hat in Reihe zum ersten Schalter 72 einen Einbahnstrompfad 81 sowie einen parallel zum Einbahnstrompfad geschalteten Parallelstrompfad 82. Im Einbahnstrompfad 81 ist das Ansteuerelement 52 in Reihe zum ersten Schalter 32 geschaltet. Der Einbahnstrompfad 81 weist mit wenigstens einem Bauteil mit Diodenfunktion auf, so dass ein Strom durch den Einbahnstrompfad 81 nur in eine Richtung fließen kann und beispielsgemäß vom Signalanschluss 48 zum Masseanschluss 47, sofern der erste Schalter 32 geschlossen ist. Bei dem hier dargestellten Ausführungsbeispiel ist in Reihe zu dem Ansteuerelement 52 ein zusätzliches Bauteil mit Diodenfunktion geschaltet, beispielsgemäß eine zweite Diode 83. Die Anode der zweiten Diode 83 ist mit dem Signalanschluss 48 verbunden und die Kathode der zweiten Diode 83 ist mit dem Ansteuerelement 52 verbunden und beispielsgemäß mit der Anode der das Ansteuerelement 52 bildenden Leuchtdiode.

Außerdem ist der Verbindungspunkt zwischen der zweiten Diode 83 und dem Ansteuerelement 52 bei dem veranschaulichten Ausführungsbeispiel über einen Verbindungsstrompfad 84 mit dem zweiten Schalter 33 verbunden, so dass der Verbindungsstrompfad 84 und der zweite Schalter 33 parallel zum Ansteuerelement 52 und dem ersten Schalter 32 geschaltet sind.

Im Parallelstrompfad 82 ist parallel zur zweiten Diode 83 und dem Ansteuerelement 72 ein vierter Widerstand 85 geschaltet.

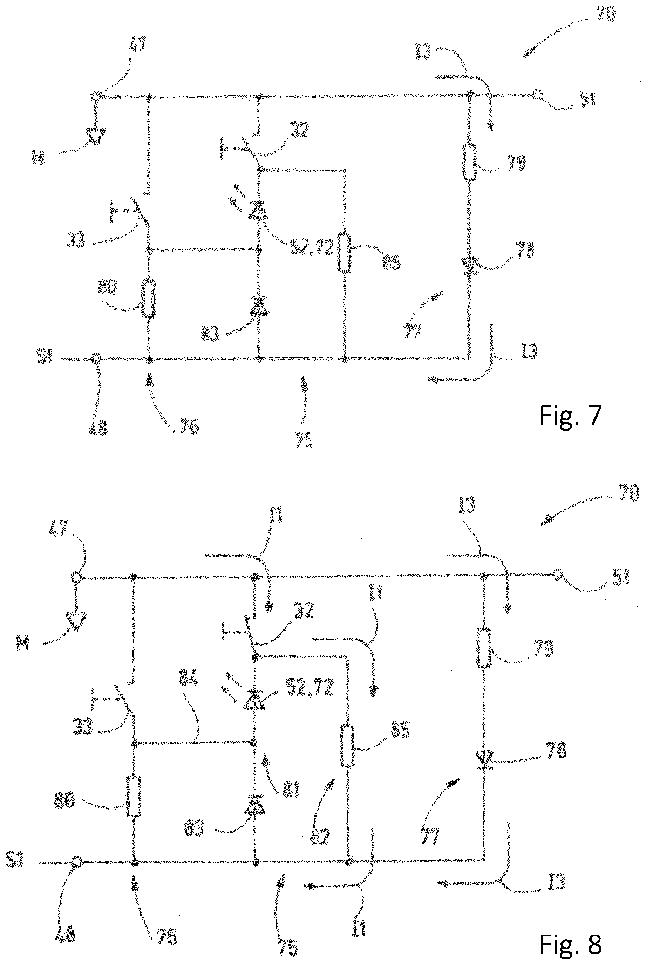

Die Funktionsweise der Betriebsschaltung 45 wird nachfolgend anhand der Figuren 4 bis 12 erläutert. Zur Unterscheidung wird dabei ein durch den ersten Schaltungszweig 75 fließender Strom als erster Strom I1, ein durch den zweiten Schaltungszweig 76 fließender Strom als zweiter Strom I2 und ein durch den dritten Schaltungszweig 77 fließender Strom als dritter Strom I3 bezeichnet (Figuren 7-12). Die Widerstandswerte der Widerstände 79, 85 und 80 in den Schaltungszweigen 75, 76, 77 werden so gewählt, dass die Auswerteschaltung 40 des Geräts 21 am Betrag der Ströme I1, I2, I3 erkennt, ob der erste Schalter 32, der zweite Schalter 33 oder beide Schalter 32, 33 im leitenden Zustand ist bzw. sind. Insbesondere sind die Widerstandswerte des dritten Widerstands 80 und des vierten Widerstands 85 unterschiedlich groß.

Die Steuerschaltung 70 ist dazu eingerichtet, eine Eigenschaft des von der Auswerteschaltung 40 bereitgestellten Auswertesignals S zu beeinflussen. Beim Ausführungsbeispiel wird hierfür der Betrag eines Auswertestromes, der zwischen dem zweiten Geräteausgang 24 und dem dritten Geräteausgang 23 fließt, durch die Steuerschaltung 70 abhängig davon verändert, in welchem Schaltzustand sich die beiden Schalter 32, 33 befinden. Die Auswerteschaltung 40 kann den Betrag des fließenden Auswertestromes zwischen dem zweiten Geräteausgang 24 und dem dritten Geräteausgang 23 (Masse M) messen, beispielsweise mit Hilfe eines Messwiderstands.

Diese Möglichkeit, den Betrag des Auswertestromes durch die Steuerschaltung 70 zu variieren, erlaubt eine einfache Erkennung des Schaltzustandes des ersten Schalters 32 und des zweiten Schalters 33. Alternativ dazu könnten auch andere Eigenschaften des Auswertesignals S verändert werden abhängig davon, durch welchen der Schaltungszweige 75, 76 ein Strom fließt, beispielsweise durch Ändern des Spannungsniveaus mittels unterschiedlich dimensionierter Zenerdioden in den Schaltungszweigen 75, 76 oder durch aktive Bauelemente in den Schaltungszweigen, die die Spannung bzw. den Strom unterschiedlich modulieren, usw.

In allen Fällen ist es der Auswerteschaltung 40 des Geräts 21 möglich, anhand der durch die Steuerschaltung 70 eingestellten Eigenschaft des Auswertesignals S (beispielsgemäß des Betrages des Auswertestromes) zu erkennen, ob der erste Schalter 32 und/oder der zweite Schalter 33 betätigt und dadurch in den leitenden Zustand umgeschaltet wurden, so dass erkannt werden kann, ob eine Bedienperson einen Dissektionsmodus oder einen Koagulationsmodus anfordert. Durch Betätigen des ersten Schalters 32 wird der Dissektionsmodus und durch Betätigen des zweiten Schalters 33 der Koagulationsmodus angefordert.

Beim Ausführungsbeispiel ist das Auswertesignal S in einem Ausgangszustand konstant und weist ausschließlich die beispielsgemäß negative erste Polarität S1 auf, wie es schematisch in Figur 4 gezeigt ist.

Im Ausgangszustand des Auswertesignals S sei angenommen, dass beide Schalter 32, 33 unbetätigt und daher nicht leitend sind (Figur 7). Wegen des negativen Potentials am Signalanschluss 48 kann lediglich ein Stromfluss vom Masseanschluss 47 zum Signalanschluss 48 erfolgen. Der erste Schaltungszweig 75 und der zweite Schaltungszweig 76 sind aufgrund der nicht leitenden Schalter 32 und 33 unterbrochen. Es kann daher nur ein dritter Strom I3 durch den dritten Schaltungszweig 77 fließen. Der Betrag des dritten Stromes I3 wird in der Auswerteschaltung 40 ausgewertet und dadurch erkannt, dass beide Schalter 32, 33 unbetätigt und daher nicht leitend sind.

Es sei angenommen, dass die Bedienperson den ersten Schalter 32 zur Anforderung des Dissektionsmodus zu einem ersten Zeitpunkt t1 betätigt, so dass dieser in seinen leitenden Zustand umgeschaltet wird (Figur 8). Zusätzlich zu dem durch den dritten Schaltungszweig 77 fließenden dritten Strom 13, kann jetzt ein erster Strom I1 über den ersten Schalter 32 und durch den vierten Widerstand 85 im Parallelstrompfad 82 fließen. Der Gesamtwiderstand der Steuerschaltung 70 ergibt sich somit im Wesentlichen aus der Parallelschaltung des zweiten Widerstands 79 und des vierten Widerstands 85. Der Betrag des Auswertestromes entspricht der Summe aus den Beträgen des ersten Stromes I1 und des zweiten Stromes 13, der sich gegenüber dem unbetätigten Zustand (Figur 7) ändert. Diese Änderung kann in der Auswerteschaltung 40 erfasst werden.

Es wird somit im Gerät 21 erkannt, dass eine Bedienperson den ersten Schalter 32 betätigt hat. Die Auswerteschaltung 40 ist dazu eingerichtet, in diesem Fall den Dissektionsmodus zu starten und das Instrument 26 entsprechend zu steuern. Zunächst wird zum ersten Zeitpunkt t1 über das Aktivierungssignal W die Versorgungsspannung UV am ersten Geräteausgang 22 bereitgestellt, so dass zwischen dem ersten Koagulationsausgang 50 und dem zweiten Koagulationsausgang 51 eine Koagulationsspannung UK anliegt, die von der HF-Spannungsquelle 42 bereitgestellt wird (Figur 5) .

Mit Beginn des ersten Zeitpunkts t1, zu dem der erste Schalter 32 betätigt wurde, startet eine erste Phase P1 im Dissektionsmodus. Während dieser ersten Phase P1 behält das Auswertesignal S die erste Polarität S1 bei, wodurch die Umschalteinheit 53 ihren zweiten Schaltzustand einnimmt. Am Schneidausgang 54 liegt dann keine Schneidspannung US bzw. kein Schneidstrom an. Beispielsgemäß sind Spannung und Strom im zweiten Schaltzustand der Umschalteinheit 53 am Schneidausgang 54 im Wesentlichen gleich Null. Bei einer abgewandelten Ausführungsform könnte die Umschalteinheit 53 im zweiten Schaltzustand eine Verbindung zwischen dem Versorgungsanschluss 46 und dem Schneidausgang 54 herstellen, in der der Transformator 60 überbrückt ist, so dass am Schneidausgang 54 ebenfalls die Koagulationsspannung UK anliegt.

Die erste Phase P1 endet, wenn eine Bedingung für das Beenden der ersten Phase P1 eintritt, beispielsweise der Ablauf einer vorgegebenen Zeitspanne. Zusätzlich oder alternativ kann ein durch die Koagulationsspannung UK bewirkter Koagulationsstrom durch das Gerät 21 ausgewertet werden und die erste Phase P1 beendet werden, wenn der Koagulationsstrom einen Schwellenwert unterschreitet.

Nach dem Ende der ersten Phase P1 (zweiter Zeitpunkt t2) startet unmittelbar eine zweite Phase P2. In dieser zweiten Phase P2 wird eine Schneidspannung US an den Schneidausgang 54 angelegt. Dazu muss die Umschalteinheit 53 vom zweiten Schaltzustand in den ersten Schaltzustand umgeschaltet werden. Dies wird beispielsgemäß dadurch erreicht, dass die Auswerteschaltung 40 das Auswertesignal S während der zweiten Phase P2 ändert, so dass es wechselnde Polaritäten S1, S2 aufweist. Das Auswertesignal S kann während dieser zweiten Phase P2 eine Wechselspannung mit positiven und negativen Halbwellen sein. Die Amplitude der positiven und der negativen Halbwellen kann gleich oder unterschiedlich groß sein. Die Zeitdauer der positiven Halbwellen und der negativen Halbwellen kann gleich oder unterschiedlich lang sein.

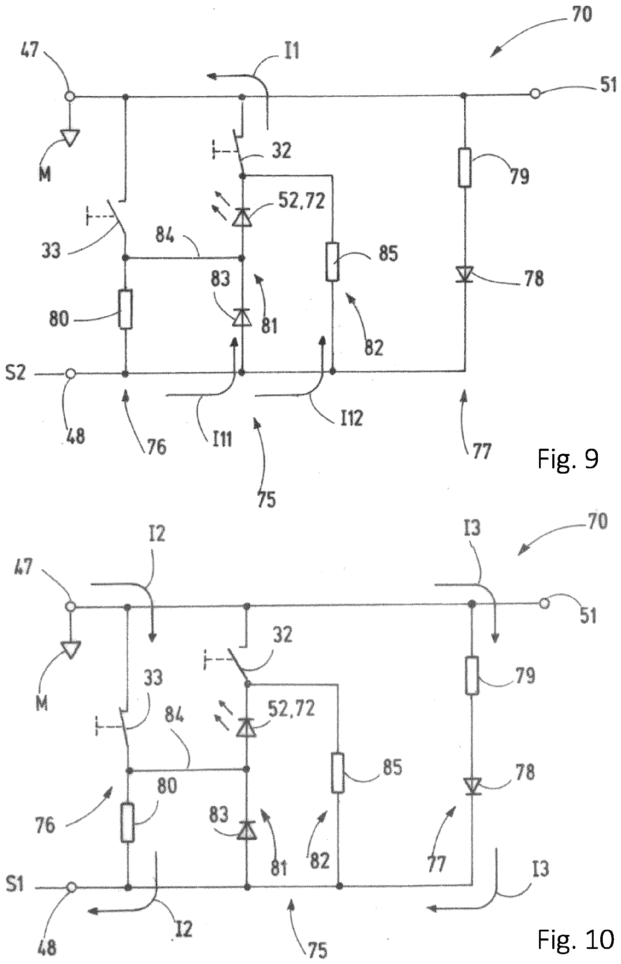

Während der positiven Halbwellen, wenn das Auswertesignal S die zweite Polarität S2 aufweist, wird in der Steuerschaltung 70 die Stromflussrichtung umgekehrt und der Auswertestrom fließt vom Signalanschluss 48 zum Masseanschluss 47 (Figur 9). Der zweite Schaltungszweig 76 ist aufgrund des nicht betätigten zweiten Schalters 33 unterbrochen und ein Stromfluss durch den dritten Schaltungszweig 77 ist wegen der ersten Diode 78 in Richtung zum Masseanschluss 47 nicht möglich. Es fließt lediglich ein erster Strom I1 durch den ersten Schaltungszweig 75.

Der erste Strom I1 weist dabei einen Teilstrom I11 durch den Einbahnstrompfad 81 und einen Teilstrom I12 durch den Parallelstrompfad 82 auf. Der Teilstrom I11 durch den Einbahnstrompfad 81 ist wegen des geringeren Widerstandswertes verglichen mit dem Widerstandswert des vierten Widerstands 85 deutlich größer als der zweite Teilstrom I12 durch den Parallelstrompfad 82. Der Teilstrom I11 fließt durch das Ansteuerelement 52, das beim Ausführungsbeispiel gleichzeitig das Senderbauteil 72 bildet. Dadurch wird das Ansteuersignal A erzeugt und an das Empfängerbauteil 73 übermittelt. Daraufhin veranlasst das Empfängerbauteil 73 das Umschalten der Umschalteinheit 53 vom zweiten Schaltzustand in den ersten Schaltzustand. In diesem ersten Schaltzustand ist der wenigstens eine Halbleiterschalter 55 leitend, so dass eine elektrische Verbindung zwischen den Schaltanschlüssen 57 der Umschalteinheit 53 hergestellt ist. Eine elektrische Verbindung zwischen dem Versorgungsanschluss 46 und dem Mittelabgriff 63 des Transformators 60 ist hergestellt. Diese elektrische Verbindung sorgt dafür, dass die am Versorgungsanschluss 46 anliegende Versorgungsspannung UV über den Transformator 60 in eine Schneidspannung US transformiert wird, die am Schneidausgang 54 anliegt (Figur 6).

Das Ansteuersignal A wird beispielsgemäß durch das von der Leuchtdiode (Senderbauteil 72) des Optokopplers 74 übertragene Licht gebildet. Die Leuchtdiode des Optokopplers 74 strahlt nur dann Licht ab, wenn ein Strom durch die Leuchtdiode fließt. Dieses abgestrahlte Licht (Ansteuersignal A) sorgt wiederum dafür, dass die wenigstens eine Fotodiode des Optokopplers 74 eine Spannung erzeugt, die als Quelle zur Bildung einer Drain-Source-Spannung oder Basis-Emitter-Spannung durch die Lade- und Entladeschaltung 58 dient, so dass der wenigstens eine Halbleiterschalter 55 in den leitenden Zustand umgeschaltet bzw. im leitenden Zustand gehalten werden kann.

Die zweite Phase P2 endet, wenn eine Bedingung zum Beenden der zweiten Phase P2 eingetreten ist. Dies kann beispielsweise der Ablauf einer vorgegebenen Zeitdauer für die zweite Phase P2 sein. Die Bedingung zum Beenden der zweiten Phase P2 kann auch erfüllt sein, wenn ein Schneidstrom, der vom Schneidausgang 54 durch das Gewebe fließt, einen Schwellenwert unterschritten hat, was durch das Gerät 21 ausgewertet werden kann.

Wie es in Figur 6 veranschaulicht ist, endet die zweite Phase P2 zu einem dritten Zeitpunkt t3. Zu diesem Zeitpunkt t3 beginnt eine dritte Phase P3. Während der dritten Phase P3 erzeugt die Auswerteschaltung 40 ein Auswertesignal S, das dem Ausgangszustand entspricht und mithin lediglich eine erste Polarität S1 aufweist, beispielsgemäß mit einem konstanten Betrag. Die dritte Phase P3 endet, wenn eine Bedingung zum Beenden der dritten Phase P3 eingetreten ist, beispielsweise wenn eine vorgegebene Zeitdauer abgelaufen ist.

Während der dritten Phase P3 bleibt die Koagulationsspannung UK zwischen den beiden Koagulationsausgängen 50, 51 erhalten. Nach Ablauf der dritten Phase P3 (vierter Zeitpunkt t4) schaltet die Auswerteschaltung 40 die Versorgungsspannung UV über das Aktivierungssignal W ab. Die Dissektion ist vollständig abgeschlossen.

Die dritte Phase P3 ist optional und kann auch entfallen.

Da das Auswertesignal S während jeder der Phasen P1, P2, P3 auch zumindest während eines Zeitabschnitts die erste Polarität S1 aufweist, kann dieser wenigstens eine Zeitabschnitt dazu verwendet werden, zu erkennen, ob der erste Schalter 32 nach wie vor betätigt ist. Sollte die Bedienperson vor Abschluss der Dissektion den ersten Schalter 32 lösen, wird dies erkannt und über das Aktivierungssignal W wird die Versorgungsspannung UV für das Instrument 26 abgeschaltet. Der Schneidvorgang kann somit in jeder Phase P1, P2, P3 der Dissektion unterbrochen werden.

In Figur 10 ist beispielhaft die Situation veranschaulicht, dass die Bedienperson den zweiten Schalter 33 betätigt, solange ein Auswertesignal S im Ausgangszustand (ausschließlich erste Polarität S1) anliegt. Dadurch wird ein Auswertestrom enthaltend einen zweiten Strom I2 durch den zweiten Schaltungszweig 76 sowie einen dritten Strom I3 durch den dritten Schaltungszweig 77 erzeugt (Figur 10). Wie bereits erläutert, kann die Auswerteschaltung 40 den Betrag des Auswertestromes aus der Summe des zweiten Stromes I2 und des dritten Stromes I3 erfassen und erkennen, dass der zweite Schalter 33 betätigt wurde. Der Gesamtwiderstand der Steuerschaltung 70 ergibt sich in dieser Situation im Wesentlichen durch die Parallelschaltung des zweiten Widerstands 79 und des dritten Widerstands 80.

Das Schließen des zweiten Schalters 33 signalisiert, dass die Bedienperson den Koagulationsmodus anfordert. Unter Verweis auf die Figuren 4-6 sei beispielhaft angenommen, dass der zweite Schalter 33 zum ersten Zeitpunkt t1 betätigt wurde. Analog zum Dissektionsmodus wird über das Aktivierungssignal W durch die Auswerteschaltung 40 das Anlegen der Versorgungsspannung UV am ersten Geräteausgang 22 veranlasst, so dass die Koagulationsspannung UK an den ersten Koagulationsausgang 50 angelegt wird (Figur 5). Das Auswertesignal S wird bei der Anforderung des Koagulationsmodus nicht verändert und behält seinen Ausgangszustand bei. Dadurch bleibt die Umschalteinheit 53 in ihrem zweiten Schaltzustand und es wird keine Schneidspannung US an den Schneidausgang 54 angelegt (strichpunktierte Linie in Figur 6).

Analog zum Dissektionsmodus kann auch im Koagulationsmodus das Erzeugen der Koagulationsspannung UK an eine Beendigungsbedingung geknüpft sein und beispielsweise durch Auswertung des Koagulationsstromes oder nach Zeitablauf beendet werden, wie es im Zusammenhang mit dem Dissektionsmodus bereits erläutert wurde.

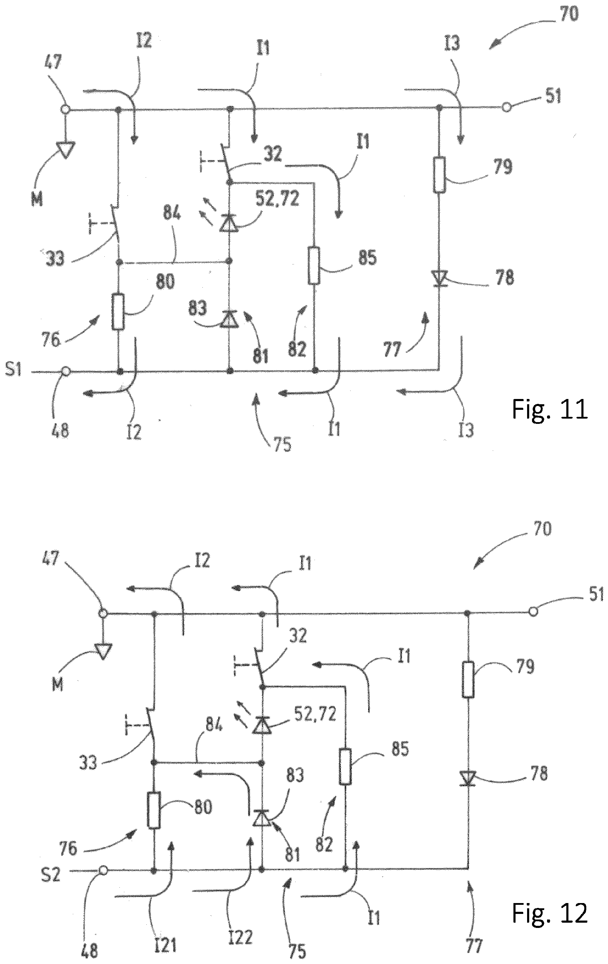

In Figur 11 ist eine Situation veranschaulicht, in der die Bedienperson sowohl den ersten Schalter 32, als auch den zweiten Schalter 33 betätigt, wenn das Auswertesignal S seinen Ausgangszustand mit ausschließlich erster Polarität S1 aufweist. Der Betrag des Auswertestromes besteht in diesem Fall aus der Summe der Beträge des ersten Stromes I1 durch den ersten Schaltungszweig 75, des zweiten Stromes I2 durch den zweiten Schaltungszweig 76 sowie des dritten Stromes I3 durch den dritten Schaltungszweig 77. Der Gesamtwiderstand der Steuerschaltung 70 ergibt sich im Wesentlichen durch die Parallelschaltung des zweiten Widerstands 79, des dritten Widerstands 80 und des vierten Widerstands 85. Die Auswerteschaltung 40 des Geräts 21 kann somit erkennen, dass beide Schalter 32, 33 betätigt wurden, indem der Betrag des Auswertestromes ausgewertet wird.

Eine Möglichkeit in einem solchen Fall besteht darin, dass das Auswertesignal S einen Zustand ausschließlich mit erster Polarität S1 beibehält und lediglich der Koagulationsmodus aktiviert wird, wie er vorstehend beschrieben wurde. Wenn das Auswertesignal S keine Abschnitte mit zweiter Polarität S2 aufweist, wird kein Ansteuersignal A generiert, das die Umschalteinheit 53 in den ersten Schaltzustand umschalten könnte. Das Erzeugen der Schneidspannung US zur Dissektion unterbleibt.

Selbst wenn in Analogie zum Dissektionsmodus das Auswertesignal S eine Phase mit wechselnder Polarität S1, S2 aufweist, wird das Umschalten der Umschalteinheit 53 in den ersten Schaltzustand unterbunden (vergleiche Figur 12). Dadurch, dass der zweite Schalter 33 das Ansteuerelement 52 überbrückt, wird ein Stromfluss durch das Ansteuerelement 52 vermieden und dadurch kein Ansteuersignal A generiert, dass das Umschalten der Umschalteinheit 53 in den ersten Schaltzustand veranlasst. Auch dann wird das Erzeugen einer Schneidspannung US vermieden.

Das wenigstens eine Empfängerbauteil 73 ist mit dem Steueranschluss 56 jedes Halbleitschalters 55 über die Lade- und Entladeschaltung 58 verbunden, um einerseits die Ladungen in den Steueranschlüssen 56 ausreichend lange halten zu können (zumindest während der Zeitdauer einer ersten Halbwelle mit der ersten Polarität S1) und andererseits die dort vorhandenen Ladungen auch wieder abführen zu können, wenn die Umschalteinheit 53 in den zweiten Schaltzustand umgeschaltet werden soll.

Über die Lade- und Entladeschaltung 58 wird die Ladung beim Ausführungsbeispiel in den Gate-Anschlüssen der MOSFETs aufrecht erhalten, so dass die Umschalteinheit 53 in ihrem zweiten Schaltzustand (leitender Zustand) bleibt, auch wenn während der zweiten Phase P2 eine Halbwelle mit erster Polarität S1 anliegt. Zumindest für die Dauer einer Halbwelle mit erster Polarität S1 wird die Ladung in den Gates der MOSFETs durch die Lade- und Entladeschaltung 58 aufrecht erhalten, wenn die Fotodioden des Optokopplers 74 bei leitendem ersten Schalter 32 während einer zweiten Halbwelle S2 erneut durch die Leuchtdiode des Optokopplers 74 angesteuert werden.

In Figur 13 ist ein Ausführungsbeispiel einer Lade- und Entladeschaltung 58 veranschaulicht. Es versteht sich, dass auch andere Lade- und Entladeschaltungen verwendet werden können.

Das wenigstens einen Empfängerbauteil 73 hat einen ersten Anschluss 73a höheren elektrischen Potenzials (hier: Anodenseite der wenigstens einen Fotodiode) und einen zweiten Anschluss 73b niedrigeren elektrischen Potenzials (hier: Kathodenseite der wenigstens einen Fotodiode). Am ersten Anschluss 73a liegt bei einer Ansteuerung durch das Senderbauteil 72 ein höheres Potenzial an als am zweiten Anschluss 73b.

Parallel zum wenigstens einen Empfängerbauteil 73 ist ein fünfter Widerstand 90 geschaltet. An den ersten Anschluss 73a ist eine Anode einer dritten Diode 91 angeschlossen, deren Kathode mit einem vierten Kondensator 92 verbunden ist. Die andere Seite des vierten Kondensators 92 ist mit dem zweiten Anschluss 73b verbunden. Der fünfte Widerstand 90 ist parallel zu der Reihenschaltung aus der dritten Diode 91 und dem vierten Kondensator 92 geschaltet.

Eine Reihenschaltung aus einer vierten Diode 93 und einem fünften Kondensator 94 ist parallel zur dritten Diode 91 geschaltet, wobei die Anode der vierten Diode 93 mit der Kathode der dritten Diode 91 verbunden ist. Die Kathode der vierten Diode 93 ist mit einer Anode einer fünften Diode 95 verbunden. Die Kathode der fünften Diode 95 ist mit einem sechsten Kondensator 96 verbunden. Der andere Anschluss des sechsten Kondensators 96 ist mit dem zweiten Anschluss 73b verbunden. Außerdem ist der sechste Kondensator 96 zwischen die Steueranschlüsse 56 (Drain-Anschlüsse) und den Verbindungspunkt zwischen den beiden in Reihe geschalteten Halbleiterschaltern 55 (Source-Anschlüsse) geschaltet. Parallel zum sechsten Kondensator 96 ist ein sechster Widerstand 97 geschaltet.

Die mehreren Kaskaden aus jeweils einer Diode 91, 93, 95 und einem in Reihe geschalteten Kondensator 92, 94, 96 dienen zur Spannungsvervielfachung der an dem wenigstens einen Empfängerbauteil 73 bei der Ansteuerung durch das Senderbauteil 72 anliegenden Spannung. Durch die vorhandenen Kondensatoren kann die Ansteuerung der Halbleitschalter 55 und beispielsgemäß die Ladung in den Gates der Feldeffekttransistoren aufrechterhalten werden, auch wenn während einer Halbwelle mit erster Polarität S1 kurzzeitig keine Spannung an dem wenigstens einen Empfängerbauteil 73 anliegt. Die Kondensatoren dienen somit als Pufferkondensatoren. Um ein Entladen zu ermöglichen, sind die Widerstände 90, 97 der Lade- und Entladeschaltung 58 vorhanden. Wenn der erste Schalter 32 unbetätigt ist, können sich die Ladungen in den Ansteueranschlüssen 56 über die Widerstände 90, 97 ausgleichen und die Halbleitschalter 55 können in ihren sperrenden Zustand zurückkehren. Die Zeitdauer ab dem Umschalten des ersten Schalters 32 in den nicht leitenden Zustand bis zum Sperren der Halbleitschalter 55 hängt von der Dimensionierung der in der Lade- und Entladeschaltung 58 vorhandenen Bauteile ab.

In Abwandlung zu der vorstehend beschriebenen Lade- und Entladeschaltung 58 können auch mehr oder weniger Kaskaden von Dioden und Kondensatoren verwendet werden. Dies hängt davon ab, welche Spannung für das Durchsteuern der Halbleiterschalter 55 benötigt wird.

In Figur 14 ist ein abgewandeltes Ausführungsbeispiel der Umschalteinheit 53 veranschaulicht. Die Halbleiterschalter 55 weisen Bipolartransistoren 103 auf und werden jeweils durch einen Halbleitersteuerschalter 102 angesteuert. Die Halbleitersteuerschalter 102 sind durch Feldeffekttransistoren beispielsgemäß durch normal sperrende n-Kanal MOSFETS gebildet. Die Steueranschlüsse 56 der Halbleitsteuerschalter 102 sind mit der Lade- und Entladeschaltung 58 verbunden. Außerdem sind die beiden Kollektoren der Bipolartransistoren 103 mit der Lade- und Entladeschaltung 58 verbunden. Jeder Emitter eines Bipolartransistors 103 bildet einen Schaltanschluss 57. Die Kollektoren der Bipolartransistoren 103 sind außerdem mit den Source-Anschlüssen, der die Halbleitersteuerschalter 102 bildenden Feldeffekttransistoren verbunden. Die Basis jedes Bipolartransistors 103 ist mit dem Drain-Anschluss eines jeweils zugeordneten Feldeffekttransistors verbunden. Sobald die Gate-Source-Spannung ausreichend groß ist, werden die Halbleitersteuerschalter 102 leitend, so dass ein Basisstrom aus den PNP-Bipolaransistoren 103 fließt und diese in ihren leitenden Zustand übergehen.

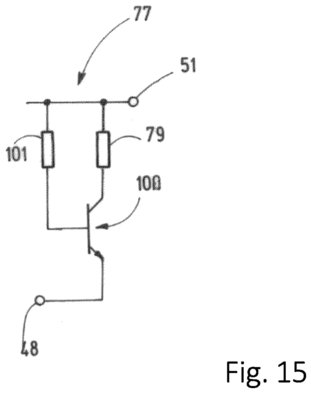

In Figur 15 ist eine abgewandelte Ausführungsform des dritten Schaltungszweiges 77 veranschaulicht anstelle der ersten Diode 78 kann ein Transistor 100, insbesondere Bipolartransistor verwendet werden, dessen Kollektoranschluss mit dem zweiten Widerstand 79 verbunden ist und dessen Emitteranschluss mit dem Signalanschluss 48 verbunden ist. Der Basisanschluss des Transistors 100 ist über einen siebten Widerstand 101 mit Masse verbunden.

Die Erfindung betrifft eine Vorrichtung zur Koagulation und/oder Dissektion von biologischen Gewebe und ein Verfahren zum Betreiben einer Vorrichtung. Die Vorrichtung 20 weist ein Gerät 21 und ein elektrisch mit dem Gerät 21 verbundenes Instrument 26 auf. An dem Instrument 26 ist zumindest ein erster Schalter 32 zur manuellen Betätigung vorhanden, der zu einer Betriebsschaltung 45 gehört. Eine Auswerteschaltung 40 des Geräts 21 stellt ein Auswertesignal S für das Instrument 26 bereit. Außerdem kann das Gerät 21 auf Anforderung eine Versorgungsspannung UV oder alternativ einen Versorgungsstrom IV erzeugen und für das Instrument 26 bereitstellen. Im Ausgangszustand weist das Auswertesignal S lediglich eine Polarität auf und ist im Ausgangszustand entweder immer größer oder gleich Null oder entweder immer kleiner oder gleich Null, aber nicht kontinuierlich, sondern allenfalls zeitweise gleich Null. Das Auswertesignal S wird einer Steuerschaltung 70 des Instruments 26 bereitgestellt, in der eine Eigenschaft des Auswertesignals S abhängig vom Betätigungszustand des ersten Schalters 32 eingestellt wird. Die Auswerteschaltung 40 des Geräts 21 detektiert diese Eigenschaft und kann daraufhin erkennen, ob eine Bedienperson über den ersten Schalter 32 den Dissektionsmodus aktiviert. Daraufhin kann die Auswerteschaltung 40 das Gerät 21 veranlassen, eine Versorgungsspannung UV oder einen Versorgungsstrom IV für das Instrument 26 bereitzustellen und das Auswertesignal S wird entsprechend dem angeforderten Dissektionsmodus verändert, insbesondere im Hinblick auf seine Polarität S1, S2. Im Dissektionsmodus weist das Auswertesignal S zumindest zeitweise auch eine zweite Polarität S2 auf.

### Bezugszeichenliste:

- 20: Vorrichtung zur Koagulation und Dissektion
- 21: Gerät
- 22: erster Geräteausgang
- 23: dritter Geräteausgang
- 24: zweiter Geräteausgang
- 25: Kabel
- 26: Instrument
- 27: Gehäuse
- 28: Handgriff
- 29: Werkzeug
- 30: Verbindungsteil
- 31: Bedienelement
- 32: erster Schalter
- 33: zweiter Schalter
- 34: Branche
- 35: Branche
- 36: erste Koagulationselektrode
- 37: zweite Koagulationselektrode
- 38: Schneidelektrode
- 40: Auswerteschaltung
- 41: Gelenk
- 42: Spannungsquelle

- 45: Betriebsschaltung
- 46: Versorgungsanschluss
- 47: Masseanschluss
- 48: Signalanschluss
- 49: erster Kondensator
- 50: erster Koagulationsausgang
- 51: zweiter Koagulationsausgang
- 52: Ansteuerelement
- 53: Umschalteinheit
- 54: Schneidausgang
- 55: Halbleiterschalter
- 56: Steueranschluss
- 57: Schaltanschluss
- 58: Lade- und Entladeschaltung

- 60: Transformator
- 61: Primärwicklung
- 62: Sekundärwicklung
- 63: Mittelabgriff
- 64: zweiter Kondensator
- 65: dritter Kondensator
- 66: erster Widerstand

- 70: Steuerschaltung
- 71: Kopplungseinrichtung
- 72: Senderbauteil
- 73: Empfängerbauteil
- 73a: erster Anschluss
- 73b: zweiter Anschluss
- 74: Optokoppler
- 75: ersten Schaltungszweig
- 76: zweiten Schaltungszweig
- 77: dritten Schaltungszweig
- 78: erste Diode
- 79: zweiten Widerstand
- 80: dritter Widerstand
- 81: Einbahnstrompfad
- 82: Parallelstrompfad
- 83: zweite Diode
- 84: Verbindungsstrompfad
- 85: vierter Widerstand

- 90: fünfter Widerstand
- 91: dritte Diode
- 92: vierten Kondensator
- 93: vierte Diode
- 94: fünfter Kondensator
- 95: fünfte Diode
- 96: sechster Kondensator
- 97: sechster Widerstand

- 100: Transistor
- 101: siebter Widerstand
- 102: Halbleitersteuerschalter
- 103: Bipolartransistor

- A: Ansteuersignal
- I: Strom
- I1: erster Strom
- I11: Teilstrom
- I12: Teilstrom
- I2: zweiter Strom
- I21: Teilstrom
- I22: Teilstrom
- I3: dritter Strom
- IV: Versorgungsstrom
- M: Masse
- P1: erste Phase
- P2: zweite Phase
- P3: dritte Phase
- S: Auswertesignal
- S1: erste Polarität
- S2: zweite Polarität
- t1: erster Zeitpunkt
- t2: zweiter Zeitpunkt
- t3: dritter Zeitpunkt
- t4: vierter Zeitpunkt
- UK: Koagulationsspannung
- US: Schneidspannung
- UV: Versorgungsspannung

## Patentansprüche

1. Vorrichtung (20) zur Koagulation und/oder Dissektion von biologischem Gewebe aufweisend ein Gerät (21) und ein elektrisch mit dem Gerät (21) verbundenes Instrument (26),
wobei das Gerät (21) dazu eingerichtet ist, an einen ersten Geräteausgang (22) eine Versorgungsspannung (UV) oder einen Versorgungsstrom (IV) bereitzustellen und wobei das Gerät (21) eine Auswerteschaltung (40) aufweist, die dazu eingerichtet ist, an einem zweiten Geräteausgang (24) ein Auswertesignal (S) bereitzustellen,
wobei eine Betriebsschaltung (45) des Instrument (26) aufweist:
- einen mit dem ersten Geräteausgang (22) verbindbaren Versorgungsanschluss (46) und einen mit dem zweiten Geräteausgang (24) verbindbaren Signalanschluss (48),
- eine mit dem Signalanschluss (48) verbundene Steuerschaltung (70), die dazu eingerichtet ist, ein von einer Polarität (S1, S2) des Auswertesignals (S) abhängiges Ansteuersignal (A) zu erzeugen, und die einen manuell betätigbaren ersten Schalter (32) aufweist,
- eine mittels des Ansteuersignals (A) ansteuerbare Umschalteinheit (53), die zwischen einem ersten Schaltzustand und einem zweiten Schaltzustand umschaltbar ist und mit dem Versorgungsanschluss (46) und einem Schneidausgang (54) verbunden ist,
wobei die Steuerschaltung (70) dazu eingerichtet ist, eine Eigenschaft des Auswertesignals (S) abhängig vom Betätigungszustand des ersten Schalters (32) einzustellen, und wobei die Auswerteschaltung (40) dazu eingerichtet ist, die Eigenschaft des Auswertesignals (S) zu erfassen und die Polarität (S1, S2) des Auswertesignals (S) abhängig von der Eigenschaft einzustellen.

2. Vorrichtung nach Anspruch 1, wobei dann, wenn die Eigenschaft des Auswertesignals (S) angibt, dass der erste Schalter (32) betätigt wurde, das Auswertesignal (S) während einer ersten Phase (P1, P3) ab dem Betätigen des ersten Schalters (32) nur eine erste Polarität (S1) aufweist und während einer zweiten Phase (P2) alternierend die erste Polarität (S1) und eine der ersten Polarität (S1) entgegengesetzte zweite Polarität (S2) aufweist.

3. Vorrichtung nach Anspruch 2, wobei die Steuerschaltung (70) dazu eingerichtet ist, die Umschalteinheit (53) mittels des Ansteuersignals (A) zur Einnahme des ersten Schaltzustand zu veranlassen, wenn das Auswertesignal (S) während der zweiten Phase (P2) zumindest zeitweise die zweite Polarität (S2) aufweist, wobei im ersten Schaltzustand am Schneidausgang (54) eine elektrische Schneidspannung (US) oder ein elektrischer Schneidstrom bereitgestellt wird, der zur Dissektion von biologischem Gewebe geeignet ist.

4. Vorrichtung nach Anspruch 2 oder 3, wobei die Steuerschaltung (70) dazu eingerichtet ist, die Umschalteinheit (53) mittels des Ansteuersignals (A) zur Einnahme des zweiten Schaltzustand zu veranlassen, wenn das Auswertesignal (S) während der ersten Phase (P1) nur die erste Polarität (S1) aufweist, wobei im zweiten Schaltzustand am Schneidausgang (54) keine elektrische Schneidspannung (US) und kein elektrischer Schneidstrom bereitgestellt wird.

5. Vorrichtung nach einem der vorhergehenden Ansprüche, wobei das Gerät (21) eine ansteuerbare Spannungsquelle (42) oder Stromquelle aufweist und die Auswerteschaltung (40) dazu eingerichtet ist, die Spannungsquelle (42) oder die Stromquelle mittels eines Aktivierungssignals (W) anzusteuern.

6. Vorrichtung nach einem der vorhergehenden Ansprüche, wobei die Steuerschaltung (70) einen manuell betätigbaren zweiten Schalter (33) aufweist.

7. Vorrichtung nach Anspruch 6, wobei die Steuerschaltung (70) dazu eingerichtet ist, die Eigenschaft des Auswertesignals (S) abhängig vom Betätigungszustand des zweiten Schalters (33) einzustellen, und wobei die Auswerteschaltung (40) dazu eingerichtet ist, die Eigenschaft des Auswertesignals (S) zu erfassen und die Polarität (S1, S2) des Auswertesignals (S) abhängig von der Eigenschaft einzustellen.

8. Vorrichtung nach Anspruch 7, wobei die Auswerteschaltung (40) dazu eingerichtet ist, das Aktivierungssignal (W) derart zu erzeugen, dass die ansteuerbare Spannungsquelle (42) oder Stromquelle am ersten Geräteausgang (22) die Versorgungsspannung (UV) oder den Versorgungsstrom (IV) bereitstellt, wenn die Eigenschaft des Auswertesignals (S) angibt, dass der erste Schalter (32) und/oder der zweite Schalter (33) betätigt wurde.

9. Vorrichtung nach Anspruch 7 oder 8, wobei die Auswerteschaltung (40) dazu eingerichtet ist, das Auswertesignal (S) aufweisend nur die erste Polarität (S1) zu erzeugen, wenn die Eigenschaft des Auswertesignals (S) angibt, dass der zweite Schalter (33) betätigt wurde.

10. Vorrichtung nach einem der vorhergehenden Ansprüche, wobei die Steuerschaltung (70) dazu eingerichtet ist, einen Betrag des Auswertesignals (S) als Eigenschaft des Auswertesignals (S) abhängig vom Betätigungszustand des ersten Schalters (32) einzustellen.

11. Vorrichtung nach einem der vorhergehenden Ansprüche, wobei das Instrument (26) ein Werkzeug (29) mit wenigstens einer Schneidelektrode (38), wenigstens einer ersten Koagulationselektrode (36) und wenigstens einer zweiten Koagulationselektrode (37) aufweist.

12. Vorrichtung nach Anspruch 11, wobei der Schneidausgang (54) mit der wenigstens einer Schneidelektrode (38) verbunden ist und wobei ein erster Koagulationsausgang (50) der Betriebsschaltung (45) mit der wenigstens einen ersten Koagulationselektrode (36) und ein zweiter Koagulationsausgang (51) der Betriebsschaltung (45) mit der wenigstens einen zweiten Koagulationselektrode (37) verbunden ist.

13. Vorrichtung nach einem der vorhergehenden Ansprüche, **dadurch gekennzeichnet, dass** der erste Schalter (32) und ein zur Erzeugung des Ansteuersignals (A) eingerichtetes Ansteuerelement (52) in einem ersten Schaltungszweig (75) in Reihe geschaltet sind, wobei der erste Schaltungszweig (75) mit dem Signalanschluss (48) verbunden ist.

14. Vorrichtung nach Anspruch 13, wobei die Steuerschaltung (70) einen manuell betätigbaren zweiten Schalter (33) aufweist, der in einem zweiten Schaltungszweig (76) angeordnet ist, wobei der zweite Schaltungszweig (76) mit dem Signalanschluss (48) verbunden ist.

15. Vorrichtung nach einem der vorhergehenden Ansprüche, **dadurch gekennzeichnet, dass** die Betriebsschaltung (45) eine Transformatorschaltung (60) aufweist, die primärseitig mit dem Versorgungsanschluss (46) und sekundärseitig mit dem Schneidausgang (54) verbunden ist.

16. Verfahren zum Betreiben einer Vorrichtung, die aufweist: ein Gerät (21) und ein elektrisch mit dem Gerät (21) verbundenes Instrument (26), wobei das Gerät (21) einen ersten Geräteausgang (22) für eine Versorgungsspannung (UV) oder einen Versorgungsstrom (IV) und eine mit einem zweiten Geräteausgang (24) verbundene Auswerteschaltung (40) aufweist, wobei das Instrument (26) eine Betriebsschaltung (45) aufweist, wobei die Betriebsschaltung (45) einen mit dem ersten Geräteausgang (22) verbindbaren Versorgungsanschluss (46), einen mit dem zweiten Geräteausgang (24) verbindbaren Signalanschluss (48), eine mit dem Signalanschluss (48) verbundene Steuerschaltung (70), einen manuell betätigbaren ersten Schalter (32) und eine Umschalteinheit (53) aufweist, die mit dem Versorgungsanschluss (46) und einem Schneidausgang (54) verbunden ist, wobei das Verfahren folgende Schritte umfasst:
- Erzeugen eines Auswertesignals (S) durch die Auswerteschaltung (40) und Bereitstellen des Auswertesignals (S) am zweiten Geräteausgang (24),
- Empfangen des Auswertesignals (S) und Einstellen einer Eigenschaft des Auswertesignals (S) abhängig vom Betätigungszustand des ersten Schalters (32) durch die Steuerschaltung (70),
- Erfassen der Eigenschaft des Auswertesignals (S) und Einstellen der Polarität (S1, S2) des Auswertesignals (S) abhängig von der erfassten Eigenschaft des Auswertesignals (S) durch die Auswerteschaltung (40),
- Erzeugen eines von der Polarität (S1, S2) des Auswertesignals (S) abhängigen Ansteuersignals (A) durch die Steuerschaltung (70),
- Ansteuern der Umschalteinheit (53) zur Einnahme eines ersten Schaltzustands oder eines zweiten Schaltzustands mittels des Ansteuersignals (A).

## Claims

1. Device (20) for coagulation and/or cutting of biological tissue comprising an apparatus (21) and an instrument (26) electrically connected with the apparatus (21),
wherein the apparatus (21) is configured to provide a supply voltage (UV) or a supply current (IV) at a first apparatus output (22) and wherein the apparatus (21) comprises an evaluation circuit (40) that is configured to provide an evaluation signal (S) at a second apparatus output (24),
wherein an operation circuit (45) of the instrument (26) comprises:
- a supply connector (46) that can be connected with the first apparatus output (22) and a signal connector (48) that can be connected with the second apparatus output (24),
- a control circuit (70) connected with the signal connector (48) that is configured to create a control signal (A) depending on a polarity (S1, S2) of the evaluation signal (S) and that comprises a manually operable first switch (32),
- a switch unit (53) that can be controlled by the control signal (A) and that can be switched between a first switch condition and a second switch condition and that is connected with the supply connector (46) and a cutting output (54),
- wherein the control circuit (70) is configured to adjust the characteristic of the evaluation signal (S) depending on the operating condition of the first switch (32) and wherein the evaluation circuit (40) is configured to detect the characteristic of the evaluation signal (S) and to adjust the polarity (S1, S2) of the evaluation signal (S) depending on the characteristic.

2. Device according to claim 1, wherein if the characteristic of the evaluation signal (S) indicates that the first switch (32) has been operated, the evaluation signal (S) comprises only a first polarity (S1) during a first phase (P1, P3) beginning with the operation of the first switch (32) and comprises the first polarity (S1) and a second polarity (S2) opposed to the first polarity (S1) in an alternating manner during a second phase (P2).

3. Device according to claim 2, wherein the control circuit (70) is configured to cause the switch unit (53) to take the first switch condition by means of the control signal (A), if during the second phase (P2) the evaluation signal (S) comprises at least temporarily the second polarity (S2), wherein an electrical cutting voltage (US) or an electrical cutting current is provided at the cutting output (54) in the first switch condition that is suitable for cutting of biological tissue.

4. Device according to claim 2 or 3, wherein the control circuit (70) is configured to cause the switch unit (53) to take the second switch condition by means of the control signal (A), if during the first phase (P1) the evaluation signal (S) comprises only the first polarity (S1), wherein no electrical cutting voltage (US) and no electrical cutting current is provided at the cutting output (54) in the second switch condition.

5. Device according to any of the preceding claims, wherein the apparatus (21) comprises a controllable voltage source (42) or current source and wherein the evaluation circuit (40) is configured to control the voltage source (42) or the current source by means of an activation signal (W).

6. Device according to any of the preceding claims, **characterized in that** the control circuit (70) comprises a manually operable second switch (33).

7. Device according to claim 6, wherein the control circuit (70) is configured to adjust the characteristic of the evaluation signal (S) depending on the operation condition of the second switch (33) and wherein the evaluation circuit (40) is configured to detect the characteristic of the evaluation signal (S) and to adjust the polarity (S1, S2) of the evaluation signal (S) depending on the characteristic.

8. Device according to claim 7, wherein the evaluation circuit (40) is configured to create the activation signal (W) such that the controllable voltage source (42) or current source provides the supply voltage (UV) or the supply current (IV) at the first apparatus output (22), if the characteristic of the evaluation signal (S) indicates that the first switch (32) and/or the second switch (33) have been actuated.

9. Device according to claim 7 or 8, wherein the evaluation circuit (40) is configured to create the evaluation signal (S) comprising only the first polarity (S1), if the characteristic of the evaluation signal (S) indicates that the second switch (33) has been actuated.

10. Device according to any of the preceding claims, wherein the control circuit (70) is configured to adjust an amount of the evaluation signal (S) as characteristic of the evaluation signal (S) depending on the operating condition of the first switch (32).

11. Device according to any of the preceding claims, wherein the instrument (26) comprises a tool (29) having at least one cutting electrode (38), at least one first coagulation electrode (36) and at least one second coagulation electrode (37) .

12. Device according to claim 11, wherein the cutting output (54) is connected with the at least one cutting electrode (38) and wherein a first coagulation output (50) of the operation circuit (45) is connected with the at least one first coagulation electrode (36) and a second coagulation output (51) of the operation circuit (45) is connected with the at least one second coagulation electrode (37).

13. Device according to any of the preceding claims, **characterized in that** the first switch (32) and a control element (52) configured for creation of the control signal (A) are connected in series in a first circuit branch (75), wherein the first circuit branch (75) is connected with the signal connector (48).

14. Device according to claim 13, wherein the control circuit (70) comprises a manually operable second switch (33) that is arranged in a second circuit branch (76), wherein the second circuit branch (76) is connected with the signal connector (48).

15. Device according to any of the preceding claims, **characterized in that** the operation circuit (45) comprises a transformer circuit (60) that is connected with the supply connector (46) on its primary side and with the cutting output (54) on its secondary side.

16. Method for operating a device comprising: an apparatus (21) and an instrument (26) that is electrically connected with the apparatus (21), wherein the apparatus (21) comprises a first apparatus output (22) for a supply voltage (UV) or a supply current (IV) and an evaluation circuit (40) connected with a second apparatus output (24), wherein the instrument (26) comprises an operation circuit (45), wherein the operation circuit (45) comprises a supply connector (46) that can be connected with the first apparatus output (22), a signal connector (48) that can be connected with the second apparatus output (24), a control circuit (70) connected with the signal connector (48), a manually operable first switch (32) and a switch unit (53) that is connected with the supply connector (46) and a cutting output (54), wherein the method comprises the following steps:
- creating an evaluation signal (S) by the evaluation circuit (40) and providing the evaluation signal (S) at the second apparatus output (24),
- receiving the evaluation signal (S) and adjusting of a characteristic of the evaluation signal (S) depending on the operating condition of the first switch (32) by the control circuit (70),
- detecting the characteristic of the evaluation signal (S) and adjusting the polarity (S1, S2) of the evaluation signal (S) depending on the detected characteristic of the evaluation signal (S) by the evaluation circuit (40),
- creating a control signal (A) depending on the polarity (S1, S2) of the evaluation signal (S) by the control circuit (70),
- controlling the switch unit (53) to take a first switch condition or a second switch condition by means of the control signal (A).

## Revendications

1. Dispositif (20) de coagulation et/ou de dissection de tissus biologiques, présentant un appareil (21) et un instrument (26) relié électriquement à l'appareil (21),
dans lequel l'appareil (21) est conçu pour fournir une tension d'alimentation (UV) ou un courant d'alimentation (IV) à une première sortie d'appareil (22), et dans lequel l'appareil (21) présente un circuit d'évaluation (40) qui est conçu pour fournir un signal d'évaluation (S) à une deuxième sortie d'appareil (24),
dans lequel un circuit de service (45) de l'instrument (26) présente :
- une borne d'alimentation (46) pouvant être reliée à la première sortie d'appareil (22) et une borne de signal (48) pouvant être reliée à la deuxième sortie d'appareil (24),
- un circuit de commande (70) qui est relié à la borne de signal (48) et est conçu pour générer un signal de commande (A) dépendant d'une polarité (S1, S2) du signal d'évaluation (S), et qui présente un premier interrupteur (32) à actionnement manuel,
- une unité de commutation (53) qui peut être commandée à l'aide du signal de commande (A) et peut être commutée entre un premier état de commutation et un deuxième état de commutation, et qui est reliée à la borne d'alimentation (46) et à une sortie de coupe (54),
dans lequel le circuit de commande (70) est conçu pour régler une propriété du signal d'évaluation (S) en fonction de l'état d'actionnement du premier interrupteur (32), et dans lequel le circuit d'évaluation (40) est conçu pour détecter la propriété du signal d'évaluation (S) et régler la polarité (S1, S2) du signal d'évaluation (S) en fonction de la propriété.

2. Dispositif selon la revendication 1, dans lequel, lorsque la propriété du signal d'évaluation (S) indique que le premier interrupteur (32) a été actionné, le signal d'évaluation (S) présente seulement une première polarité (S1) pendant une première phase (P1, P3) à partir de l'actionnement du premier interrupteur (32), et présente, pendant une deuxième phase (P2), en alternance la première polarité (S1) et une deuxième polarité (S2) opposée à la première polarité (S1).

3. Dispositif selon la revendication 2, dans lequel le circuit de commande (70) est conçu pour faire en sorte, au moyen du signal de commande (A), que l'unité de commutation (53) passe au premier état de commutation lorsque le signal d'évaluation (S) présente au moins temporairement la deuxième polarité (S2) au cours de la deuxième phase (P2), sachant que dans le premier état de commutation, une tension de coupe (US) électrique ou un courant de coupe électrique, convenant à la dissection de tissus biologiques, est fourni(e) à la sortie de coupe (54).

4. Dispositif selon la revendication 2 ou 3, dans lequel le circuit de commande (70) est conçu pour faire en sorte, au moyen du signal de commande (A), que l'unité de commutation (53) passe au deuxième état de commutation lorsque le signal d'évaluation (S) présente uniquement la première polarité (S1) au cours de la première phase (P1), sachant que dans le deuxième état de commutation, aucune tension de coupe (US) électrique ni aucun courant de coupe électrique ne sont fournis.

5. Dispositif selon l'une des revendications précédentes, dans lequel l'appareil (21) présente une source de tension (42) ou une source de courant pouvant être commandée, et le circuit d'évaluation (40) est conçu pour commander la source de tension (42) ou la source de courant au moyen d'un signal d'activation (W).

6. Dispositif selon l'une des revendications précédentes, dans lequel le circuit de commande (70) présente un deuxième interrupteur (33) à actionnement manuel.

7. Dispositif selon la revendication 6, dans lequel le circuit de commande (70) est conçu pour régler la propriété du signal d'évaluation (S) en fonction de l'état d'actionnement du deuxième interrupteur (33), et dans lequel le circuit d'évaluation (40) est conçu pour détecter la propriété du signal d'évaluation (S) et régler la polarité (S1, S2) du signal d'évaluation (S) en fonction de la propriété.

8. Dispositif selon la revendication 7, dans lequel le circuit d'évaluation (40) est conçu pour générer le signal d'activation (W) de manière à ce que la source de tension (42) ou la source de courant pouvant être commandée fournisse la tension d'alimentation (UV) ou le courant d'alimentation (IV) à la première sortie d'appareil (22), lorsque la propriété du signal d'évaluation (S) indique que le premier interrupteur (32) et/ou le deuxième interrupteur (33) a été activé.

9. Dispositif selon la revendication 7 ou 8, dans lequel le circuit d'évaluation (40) est conçu, en présentant le signal d'évaluation (S), pour produire uniquement la première polarité (S1), lorsque la propriété du signal d'évaluation (S) indique que le deuxième interrupteur (33) a été actionné.

10. Dispositif selon l'une des revendications précédentes, dans lequel le circuit de commande (70) est conçu pour régler une valeur du signal d'évaluation (S) en tant que propriété du signal d'évaluation (S), en fonction de l'état d'actionnement du premier interrupteur (32).

11. Dispositif selon l'une des revendications précédentes, dans lequel l'instrument (26) présente un outil (29) comportant au moins une électrode de coupe (38), au moins une première électrode de coagulation (36) et au moins une deuxième électrode de coagulation (37).

12. Dispositif selon la revendication 11, dans lequel la sortie de coupe (54) est reliée à l'électrode de coupe (38), au nombre d'au moins une, et dans lequel une première sortie de coagulation (50) du circuit de service (45) est reliée à la première électrode de coagulation (36), au nombre d'au moins une, et une deuxième sortie de coagulation (51) du circuit de service (45) est reliée à la deuxième électrode de coagulation (37), au nombre d'au moins une.

13. Dispositif selon l'une des revendications précédentes, **caractérisé en ce que** le premier interrupteur (32) et un élément de commande (52) conçu pour la génération du signal de commande (A) sont montés en série dans une première branche de circuit (75), la première branche de circuit (75) étant reliée à la borne de signal (48).

14. Dispositif selon la revendication 13, dans lequel le circuit de commande (70) présente un deuxième interrupteur (33) à actionnement manuel, qui est placé dans une deuxième branche de circuit (76), la deuxième branche de circuit (76) étant reliée à la borne de signal (48).

15. Dispositif selon l'une des revendications précédentes, **caractérisé en ce que** le circuit de service (45) présente un circuit transformateur (60) qui est relié à la borne d'alimentation (46), côté primaire, et à la sortie de coupe (54), côté secondaire.

16. Procédé pour faire fonctionner un dispositif qui présente : un appareil (21) et un instrument (26) relié électriquement à l'appareil (21), où l'appareil (21) présente une première sortie d'appareil (22) pour une tension d'alimentation (UV) ou un courant d'alimentation (IV) et un circuit d'évaluation (40) relié à une deuxième sortie d'appareil (24), où l'instrument (26) présente un circuit de service (45), le circuit de service (45) présentant une borne d'alimentation (46) pouvant être reliée à la première sortie d'appareil (22), une borne de signal (48) pouvant être reliée à la deuxième sortie d'appareil (24), un circuit de commande (70) relié à la borne de signal (48), un premier interrupteur (32) à actionnement manuel et une unité de commutation (53) qui est reliée à la borne d'alimentation (46) et à une sortie de coupe (54), le procédé comprenant les étapes suivantes :
- génération d'un signal d'évaluation (S) par le circuit d'évaluation (40), et délivrance du signal d'évaluation (S) à la deuxième sortie d'appareil (24),
- réception du signal d'évaluation (S) et réglage d'une propriété du signal d'évaluation (S) en fonction de l'état d'actionnement du premier interrupteur (32) par le circuit de commande (70),
- détection de la propriété du signal d'évaluation (S) et réglage de la polarité (S1, S2) du signal d'évaluation (S) en fonction de la propriété détectée du signal d'évaluation (S) par le circuit d'évaluation (40),
- génération d'un signal de commande (A) par le circuit de commande (70), en fonction de la polarité (S1, S2) du signal d'évaluation (S),
- commande de l'unité de commutation (53) afin qu'elle passe à un premier état de commutation ou un deuxième état de commutation, au moyen du signal de commande (A).
